# EUROPÄISCHE PATENTSCHRIFT

(11) **EP 1 200 390 B1**
(45) Veröffentlichungstag und Bekanntmachung des Hinweises auf die Patenterteilung: **27.08.2008**
(21) Anmeldenummer: 00940108.4
(22) Anmeldetag: 13.07.2000
(51) Int. Cl.: C07C 231/12, C07C 247/12, C07C 233/09, C07C 235/34, C07C 233/20, C07D 295/18, C07D 263/24, C07D 307/32

(54) **HERSTELLUNG VON N-SUBSTITUIERTEN 2,7-DIALKYL-4-HYDROXY-5-AMINO-8-ARYL-OCTANOYLAMIDEN**
PRODUCTION OF N-SUBSTITUTED 2,7-DIALKYL-4-HYDROXY-5-AMINO-8-ARYL-OCTANOYLAMIDES
PREPARATION DE 2,7-DIALKYLE-4-HYDROXY-5-AMINO-8-ARYLE-OCTANOYLAMIDES N-SUBSTITUES

(30) Priorität: 29.07.1999 CH 140199; 11.01.2000 CH 44002000
(43) Veröffentlichungstag der Anmeldung: 02.05.2002
(73) Patentinhaber: Speedel Pharma AG, 4051 Basel (CH)
(72) Erfinder: HEROLD, Peter, CH-4057 Basel (CH); STUTZ, Stefan, CH-4053 Basel (CH); INDOLESE, Adriano, CH-4313 Möhlin (CH)
(74) Vertreter: Dannappel, Hans-Jochen
(86) Internationale Anmeldenummer: PCT/CH2000/000384
(87) Internationale Veröffentlichungsnummer: WO 2001/009083

(56) Entgegenhaltungen:
- EP-A- 0 678 503
- PETER HEROLD ET AL.: "A Versatile and Stereocontrolled Synthesis of Hydroxyethylene Dipeptide Isosters" JOURNAL OF ORGANIC CHEMISTRY., Bd. 54, Nr. 5, 3. März 1989 (1989-03-03), Seiten 1178-1185, XP002149098 AMERICAN CHEMICAL SOCIETY. EASTON., US ISSN: 0022-3263
- G¹RARD CAHIEZ ET AL.: "Highly Stereo- and Chemoselective Iron-Catalyzed Alkenylation of Organomagnesium Compounds" SYNTHESIS, Nr. 8, August 1998 (1998-08), Seiten 1199-1200, XP002149099 STUTTGART DE in der Anmeldung erwähnt

## Beschreibung

Die Erfindung betrifft ein Verfahren zur Herstellung von N-substituierten 2,7-Dialkyl-4-hydroxy-5-amino-8-aryl-octanoylamiden und ihren physiologisch verträglichen Salzen; besonders von N-(3-Amino-2,2-dimethyl-3-oxopropyl)-2,7-di(1-methylethyl)-4-hydroxy-5-amino-8-[4-methoxy-3-(3-methoxypropoxy)phenyl]octanamiden und ihren physiologisch verträglichen Salzen, ganz besonders von 2(S),4(S),5(S),7(S)-N-(3-Amino-2,2-dimethyl-3-oxopropyl)-2,7-di(1-methylethyl)-4-hydroxy-5-amino-8-[4-methoxy-3-(3-methoxypropoxy)phenyl]octanamid und ihren physiologisch verträglichen Salzen; und neue im mehrstufigen Verfahren als Zwischenprodukte verwendete Zwischenprodukte.

In der EP-A-0 678 503 werden δ-Amino-γ-hydroxy-ω-aryl-alkancarbonsäureamide beschrieben, die Renin-hemmende Eigenschaften aufweisen und als antihypertensive Mittel in pharmazeutischen Zubereitungen verwendet werden können. Die beschriebenen Herstellungsverfahren sind hinsichtlich der Anzahl von Verfahrensstufen und Ausbeuten unbefriedigend und nicht für ein industrielles Verfahren geeignet. Nachteilig bei diesen Verfahren ist auch, dass reine Diastereomere in zu geringen Gesamtausbeuten erhältlich sind.

Es wurde nun überraschend gefunden, dass man diese Alkancarbonsäureamide sowohl in hohen Gesamtausbeuten als auch in hoher Reinheit, sowie gezielt reine Diastereomere erhält, wenn man die Doppelbindung von 2,7-Dialkyl-8-aryl-4-octenoylamiden gleichzeitig in 5-Stellung halogeniert und in 4-Stellung unter Lactonisierung hydroxyliert, das Halogen mit Azid ersetzt, das Lacton amidiert und dann das Azid in die Amingruppe überführt.

Ein erster Gegenstand der Erfindung ist ein Verfahren zur Herstellung von Verbindungen der Formel Ia, 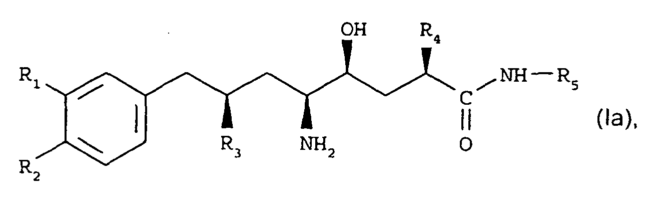 worin
R₁ und R₂ unabhängig voneinander H, C₁-C₆-Alkyl, C₁-C₆-Halogenalkyl, C₁-C₆-Alkoxy, C₁-C₆-Alkoxy-C₁-C₆-Alkyl, oder C₁-C₆-Alkoxy-C₁-C₆-Alkyloxy darstellen, R₃ C₁-C₆-Alkyl bedeutet, R₄ für C₁-C₆-Alkyl steht, und R₅ C₁-C₆-Alkyl, C₁-C₆-Hydroxyalkyl, C₁-C₆-Alkoxy-C₁-C₆-alkyl, C₁-C₆-Alkanoyloxy-C₁-C₆-alkyl, C₁-C₆-Aminoalkyl, C₁-C₆-Alkylamino-C₁-C₆-alkyl, C₁-C₆-Dialkyl-amino-C₁-C₆-alkyl, C₁-C₆-Alkanoylamido-C₁-C₆-alkyl, HO(O)C-C₁-C₆-alkyl, C₁-C₆-AlkylO-(O)C-C₁-C₆-alkyl, H₂N-C(O)-C₁-C₆-alkyl, C₁-C₆-Alkyl-HN-C(O)-C₁-C₆-alkyl oder (C₁-C₆-Alkyl)₂N-C(O)-C₁-C₆-alkyl darstellt, dadurch gekennzeichnet, dass man
a) eine Verbindung der Formel IIa 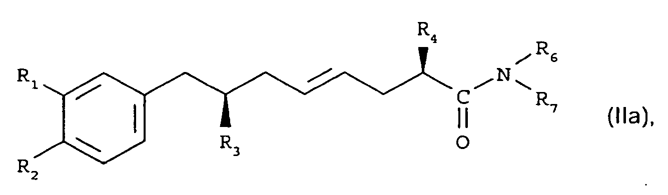 worin
   R₆ C₁-C₆-Alkyl darstellt, R₇ C₁-C₆-Alkyl oder C₁-C₆-Alkoxy bedeutet, oder R₆ und R₇ zusammen gegebenenfalls mit C₁-C₄-Alkyl, Phenyl oder Benzyl substituiertes Tetramethylen, Pentamethylen, 3-Oxa-1,5-Pentylen oder -CH₂CH₂O-C(O)- sind, mit einem Halogenierungsmittel in Gegenwart von Wasser und gegebenenfalls einer Säure zu einer Verbindung der Formel IIIa umsetzt, 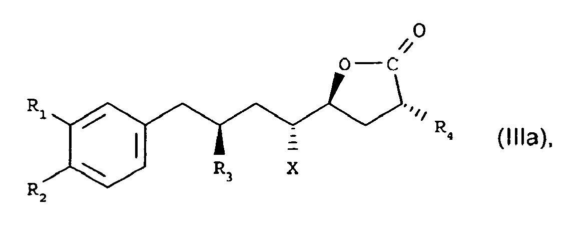 worin X für Cl, Br oder I steht,
b) die Verbindung der Formel IIIa mit einem Azidierungsmittel zu einer Verbindung der Formel IVa umsetzt, 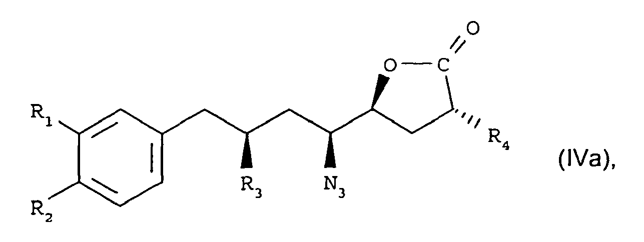
c) danach die Verbindung der Formel IVa mit einem Amin der Formel R₅-NH₂ zu einer Verbindung der Formel Va umsetzt, 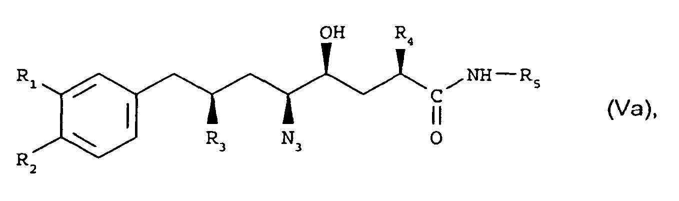 und
d) zur Herstellung der Verbindung der Formel Ia die Azidgruppe der Verbindung der Formel Va zur Amingruppe reduziert, und dann die Verbindungen der Formel Ia isoliert, gegebenenfalls unter Zugabe einer salzbildenden Säure.
R₁ und R₂ können als Alkyl linear oder verzweigt sein und bevorzugt 1 bis 4 C-Atome enthalten. Beispiele sind Methyl,
Ethyl, n- und i-Propyl, n-, i- und t-Butyl, Pentyl und Hexyl.

R₁ und R₂ können als Halogenalkyl linear oder verzweigt sein und bevorzugt 1 bis 4, besonders bevorzugt 1 oder 2 C-Atome enthalten. Beispiele sind Fluormethyl, Difluormethyl, Trifluormethyl, Chlormethyl', Dichlormethyl, Trichlormethyl, 2-Chlorethyl und 2,2,2-Trifluorethyl.

R₁ und R₂ können als Alkoxy linear oder verzweigt sein und bevorzugt 1 bis 4 C-Atome enthalten. Beispiele sind Methoxy, Ethoxy, n- und i-Propyloxy, n-, i- und t-Butyloxy, Pentyloxy und Hexyloxy.

R₁ und R₂ können als Alkoxyalkyl linear oder verzweigt sein. Die Alkoxygruppe enthält bevorzugt 1 bis 4 und besonders 1 oder 2 C-Atome, und die Alkylgruppe enthält bevorzugt 1 bis 4 C-Atome. Beispiele sind Methoxymethyl, 1-Methoxyeth-2-yl, 1-Methoxyprop-3-yl, 1-Methoxybut-4-yl, Methoxypentyl, Methoxyhexyl, Ethoxymethyl, 1-Ethoxyeth-2-yl, 1-Ethoxyprop-3-yl, 1-Ethoxybut-4-yl, Ethoxypentyl, Ethoxyhexyl, Propyloxymethyl, Butyloxymethyl, 1-Propyloxyeth-2-yl und 1-Butyloxyeth-2-yl.

R₁ und R können als C₁-C₆-Alkoxy-C₁-C₆-alkyloxy linear oder verzweigt sein. Die Alkoxygruppe enthält bevorzugt 1 bis 4 und besonders 1 oder 2 C-Atome, und die Alkyloxygruppe enthält bevorzugt 1 bis 4 C-Atome. Beispiele sind Methoxymethyloxy, 1-Methoxyeth-2-yloxy, 1-Methoxyprop-3-yloxy, 1-Methoxybut-4-yloxy, Methoxypentyloxy, Methoxyhexyloxy, Ethoxymethyloxy, 1-Ethoxyeth-2-yloxy, 1-Ethoxyprop-3-yloxy, 1-Ethoxybut-4-yloxy, Ethoxypentyloxy, Ethoxyhexyloxy, Propyloxymethyloxy, Butyloxymethyloxy, 1-Propyloxyeth-2-yloxy und 1-Butyloxyeth-2-yloxy.

In einer bevorzugten Ausführungsform bedeutet R₁ Methoxy- oder Ethoxy-C₁-C₄-Alkyloxy, und R₂ stellt bevorzugt Methoxy oder Ethoxy dar. Ganz besonders bevorzugt sind Verbindungen der Formel Ia, worin R₁ 1-Methoxyprop-3-yloxy und R₂ Methoxy bedeuten.

R₃ und R₄ können als Alkyl linear oder verzweigt sein und bevorzugt 1 bis 4 C-Atome enthalten. Beispiele sind Methyl, Ethyl, n- und i-Propyl, n-, i - und t-Butyl, Pentyl und Hexyl. In einer bevorzugten Ausführungsform stellen in den Verbindungen der Formel Ia R₃ und R₄ je Isopropyl dar.

R₅ kann als Alkyl linear oder verzweigt sein und bevorzugt 1 bis 4 C-Atome enthalten. Beispiele für Alkyl sind zuvor angegeben worden. Bevorzugt sind Methyl, Ethyl, n- und i-Propyl, n-, i- und t-Butyl.

R₅ kann als C₁-C₆-Hydroxyalkyl linear oder verzweigt sein und bevorzugt 2 bis 6 C-Atome enthalten. Einige Beispiele sind 2-Hydroxyethy-1-yl, 2-Hydroxyprop-1-yl, 3-Hydroxyprop-1-yl, 2-, 3- oder 4-Hydroxybut-1-yl, Hydroxypentyl und Hydroxyhexyl.

R₅ kann als C₁-C₆-Alkoxy-C₁-C₆-alkyl linear oder verzweigt sein. Die Alkoxygruppe enthält bevorzugt 1 bis 4 C-Atome und die Alkylgruppe bevorzugt 2 bis 4 C-Atome. Einige Beispiele sind 2-Methoxyethy-1-yl, 2-Methoxyprop-1-yl, 3-Methoxyprop-1-yl, 2-, 3- oder 4-Methoxybut-1-yl, 2-Ethoxyethy-1-yl, 2-Ethoxyprop-1-yl, 3-Ethoxyprop-1-yl, und 2-, 3- oder 4-Ethoxybut-1-yl.

R₅ kann als C₁-C₆-Alkanoyloxy-C₁-C₆-alkyl linear oder verzweigt sein. Die Alkanoylgruppe enthält bevorzugt 1 bis 4 C-Atome und die Alkylgruppe bevorzugt 2 bis 4 C-Atome. Einige Beispiele sind Formyloxymethyl, Formyloxyethyl, Acetyloxyethyl, Propionyloxyethyl und Butyroyloxyethyl.

R₅ kann als C₁-C₆-Aminoalkyl linear oder verzweigt sein und bevorzugt 2 bis 4 C-Atome enthalten. Einige Beispiele sind 2- Aminoethyl, 2- oder 3-Aminoprop-1-yl und 2-, 3- oder 4-Aminobut-1-yl.

R₅ kann als C₁-C₆-Alkylamino-C₁-C₆-alkyl und C₁-C₆-Dialkyl-amino-C₁-C₆-alkyl linear oder verzweigt sein. Die Alkylaminogruppe enthält bevorzugt C₁-C₄-Alkylgruppen und die Alkylgruppe bevorzugt 2 bis 4 C-Atome. Einige Beispiele sind 2-Methylaminoeth-1-yl, 2-Dimethylaminoeth-1-yl, sind 2-Ethyl-aminoeth-1-yl, 2-Ethylaminoeth-1-yl, 3-Methylaminoprop-1-yl, 3-Dimethylaminoprop-1-yl, 4-Methylaminobut-1-yl und 4-Dimethylaminobut-1-yl.

R₅ kann als C₁-C₆-Alkanoylamido-C₁-C₆-alkyl linear oder verzweigt sein. Die Alkanoylgruppe enthält bevorzugt 1 bis 4 C-Atome und die Alkylgruppe bevorzugt 1 bis 4 C-Atome. Einige Beispiele sind 2-Formamidoeth-1-yl, 2-Acetamidoeth-1-yl, 3-Propionylamidoeth-1-yl und 4-Butyroylamidoeth-1-yl.

R₅ kann als HO(O)C-C₁-C₆-alkyl linear oder verzweigt sein, und die Alkylgruppe enthält bevorzugt 2 bis 4 C-Atome. Einige Beispiele sind' Carboxymethyl, Carboxyethyl, Carboxypropyl und Carboxybutyl.

R₅ kann als C₁-C₆-AlkylO-(O)C-C₁-C₆-alkyl linear oder verzweigt sein, und die Alkylgruppen enhalten bevorzugt unabhängig voneinander 1 bis 4 C-Atome. Einige Beispiele sind Methoxycarbonylmethyl, 2-Methoxycarbonyleth-1-yl, 3-Methoxy-carbonylprop-1-yl, 4-Methoxycarbonylbut-1-yl, Ethoxycarbonylmethyl, 2-Ethoxycarbonyleth-1-yl, 3-Ethoxycarbonyl-prop-1-yl, 4-Ethoxycarbonylbut-1-yl.

R₅ kann als H₂N-C (O) -C₁-C₆-alkyl linear oder verzweigt sein, und die Alkylgruppe enthält bevorzugt 2 bis 6 C-Atome. Einige Beispiele sind Carbamidomethyl, 2-Carbamidoeth-1-yl, 2-Carbamido-2,2-dimethyleth-1-yl, 2- oder 3-Carbamidoprop-1-yl, 2-, 3- oder 4-Carbamidobut-1-yl, 3-Carbamido-2-methyl-prop-1-yl, 3-Carbamido-1,2 -dimethylprop-1-yl, 3-Carbamido-3-methylprop-1-yl, 3-Carbamido-2,2-dimethylprop-1-yl, 2-, 3- , 4-oder 5-Carbamidopent-1-yl, 4-Carbamido-3,3- oder -2,2-dimethylbut-1-yl.

R₅ kann als C₁-C₆-Alkyl-HN-C(O)-C₁-C₆-alkyl oder (C₁-C₆-Alkyl)₂N-C(O)-C₁-C₆-alkyl linear oder verzweigt sein, und die NH-Alkylgruppe enthält bevorzugt 1 bis 4 C-Atome, sowie die Alkylgruppe bevorzugt 2 bis 6 C-Atome. Beispiele sind die zuvor genannten Carbamidoalkylgruppen, deren N-Atom mit einem oder zwei Methyl, Ethyl, Propyl oder Butyl substituiert ist.

Eine bevorzugte Untergruppe von Verbindungen der Formel Ia ist jene, worin R₁ C₁-C₄-Alkoxy oder C₁-C₄-Alkoxy-C₁-C₄-alkyloxy bedeutet, R_{z} C₁-C₄-Alkoxy darstellt, R₃ C₁-C₄-Alkyl ist, R₄ C₁-C₄-Alkyl bedeutet und R₅ für gegebenenfalls N-mono- oder N-di-C₁-C₄-Alkyl substituiertes HNC (O) -C₁-C₆-Alkyl steht.

Eine bevorzugtere Untergruppe von Verbindungen der Formel Ia ist jene, worin R₁ Methoxy-C₂-C₄-alkyloxy bedeutet, R₂ Methoxy oder Ethoxy darstellt, R₃ C₁-C₄-Alkyl ist, R₄ C₁-C₆-Alkyl bedeutet und R₆ für H₂NC (O) -C₁-C₆-Alkyl steht.

Eine ganz besonders bevorzugte Verbindung der Formel Ia ist jene, worin R₁ 3-Methoxy-prop-3-yloxy bedeutet, R₂ Methoxy darstellt, R₃ und R₄ für 1-Methyleth-1-yl stehen, und R₅ H₂NC(O)-[C(CH₃)₂]-CH₂- darstellt.

Die Verbindungen der Formel Ia weisen 4 stereogene C-Atome auf. Erfindungsgemäss werden alle möglichen Diastereomere und beliebige Mischungen umfaßt. Bevorzugt sind die 2(S), 4(S), 5(S), 7(S)-Diastereomeren der Formel Ia 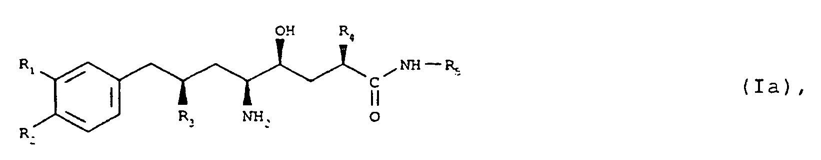 worin R₁, R₂, R₃, R₄ und R₅ die zuvor angebenen Bedeutungen haben, einschliesslich der Bevorzugungen.

Ganz besonders bevorzugt ist die Verbindung der Formel Ib

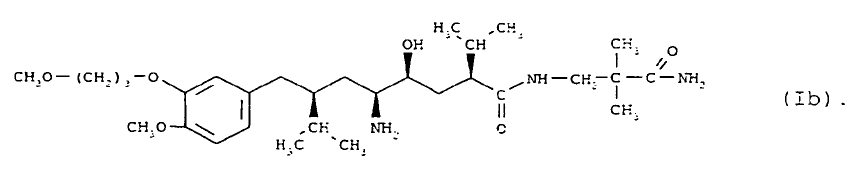

Die gewünschten Diastereomere können durch chromatographische Methoden oder fraktionierte Kristallisation aus den Gemischen isoliert oder mittels asymmetrisches Synthese erhalten werden.

R₆ und R₇ in Formel IIa können als Alkyl verzweigt und bevorzugt linear sein und bedeuten bevorzugt C₁-C₄-Alkyl, zum Beispiel Methyl oder Ethyl. R₇ kann als Alkoxy bevorzugt linear sein und ist bevorzugt C₁-C₄-Alkoxy, zum Beispiel Methoxy oder Ethoxy. R₆ und R₇ zusammen sind bevorzugt Tetramethylen, -CH₂CH₂-O-C (O) - oder -CH (CH₂C₆H₅) CH₂-O-C(O)-.

Die einzelnen Verfahrensstufen können in Gegenwart von Lösungsmittel durchgeführt werden. Geeignete Lösungsmittel sind Waser und organische Lösungsmittel, besonders polare organische Lösungsmittel, die auch als Gemische von wenigstens zwei Lösungsmitteln verwendet werden können. Beispiele für Lösungsmittel sind Kohlenwasserstoffe (Petrolether, Pentan, Hexan, Cyclohexan, Methylcyclohexan, Benzol, Toluol, Xylol), Halogenkohlenwasserstoff (Methylenchlorid, Chloroform, Tetrachlorethan, Chlorbenzol); Ether (Diethylether, Dibutylether, Tetrahydrofuran, Dioxan, Ethylenglykoldimethyl- oder diethylether); Carbonsäureester und Lactone (Essigsäuremethylester, Essigsäureethylester, Propionsäuremethylester, Valerolacton); N,N-substituierte Carbonsäureamide und Lactame (Dimethylformamid; Dimethylacetamid, N-Methylpyrrolidon); Ketone (Aceton, Methylisobutylketon, Cyclohexanon); Sulfoxide und Sulfone (Dimethylsulfoxid, Dimethylsulfon, Tetramethylensulfon); Alkohole (Methanol, Ethanol, n- oder i-Propanol, n-, i- oder t-Butanol, Pentanol, Hexanol, Cyclohexanol, Cyclohexandiol, Hydroxymethyl- oder Dihydroxymethylcyclohexan, Benzylalkohol, Ethylenglykol, Diethylenglykol, Propandiol, Butandiol, Ethylenglykolmonomethyl- oder monoethylether, und Diethylenglykolmonomethyl- oder nonoethylether; Nitile (Acetonitril, Propioniril); tertiäre Amine (Trimethyl-, Triethyl-, Tripropyl- und Tributylamin, Pyridin, N-Methylpyrrolidin, N-Methylpiperazin, N-Methylmorpholin) und organische Säuren (Essigsäure, Ameisensäure).

### Verfahrensstufe a)

Geeignete Chlorierungs-, Bromierungs und Iodierungsmittel sind elememtares Brom und Iod, insbesondere N-Chlor-, N-Brom- und N-Iodcarbonsäureamide und -dicarbonsäureimide. Bevorzugt sind N-Chlor-, N-Brom- und N-Iodphthalimid und besonders Chlor-, N-Brom- und N-Iodsuccinimid, sowie Tertiärbutylhypochlorit und N-halogenierte Sulfonamide und -imide, zum Beispiel Chloramin T. Die Reaktion wird vorteilhaft in organischen Lösungsmitteln durchgeführt. Die Reaktionstemperatur kann zum Beispiel von etwa -70 °C bis Raumtemperatur und vorzugsweise -30 °C bis 10 °C betragen. Carbonsäureamide werden vorteilhaft in Gegenwart von anorganischen oder organischen Säuren, wenigstens äquimolaren Mengen Wasser laktonisiert und wassermischbaren Lösungsmitteln, zum Beispiel Tetrahydrofuran oder Dioxan umgesetzt. Geeignete Säuren sind zum Beispiel Ameisensäure, Essigsäure, Methansulfonsäure, Trifluoressigsäure, Trifluormethansulfonsäure, Toluolsulfonsäure, H₂SO₄, H₃PO₄, Halogenwasserstoffsäuren, saure Ionenaustauschharze, auf festen Trägern immobilisierte Säuren. Wasser wird im allgemeinen in wenigstens äquimolaren Mengen eingesetzt.

Wenn man in dieser Verfahrensstufe Verbindungen der Formel IIa 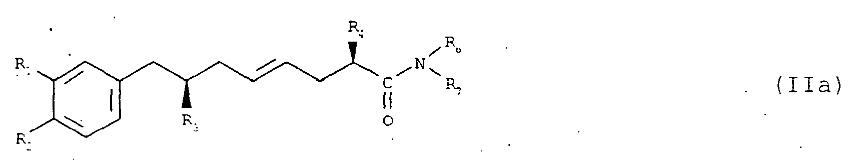 einsetzt, erhält man Verbindungen der Formel IIIa

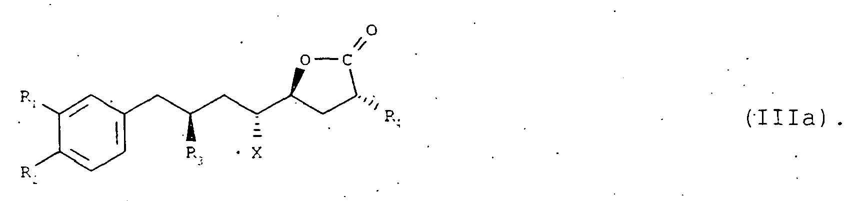

### Verfahrensstufe b)

Geeignete Azidierungsmittel sind zum Beispiel Metallazide, besonders Erdalkalimetallazide und Alkalimetallazide, sowie Silylazide. Besonders bevorzugte Azidierungsmittel sind Lithiumamid, Natriumazid und Kaliumazid. Die Reaktion kann in organischen Lösungsmitteln, vorteilhaft in wassermischbaren Lösungsmitteln in Abmischung mit Wasser durchgeführt werden, wie zum Beispiel Alkoholen oder Ethern (Methanol, Ethanol, Ethylenglykol, Diethylenglykol, Diethylenglykolmonomethyl- oder -ethylether, Diethylether, Tetrahydrofuran, Dioxan). Die Reaktionstemperatur kann zum Beispiel etwa 20 °C bis 150 °C und vorzugsweise 50 °C bis 120 °C betragen. Es kann zweckmässig sein, Phasentransferkatalysatoren mit zu verwenden. Herstellung und synthetische Verwendung von Aziden sind zum Beispiel von E. F. V. Scriven in Chemical Reviews, Vol. 88 (1988), Seiten 298 bis 317 beschrieben.

Wenn man Verbindungen der Formel IIIa einsetzt, erhält man Verbindungen der Formel IVa

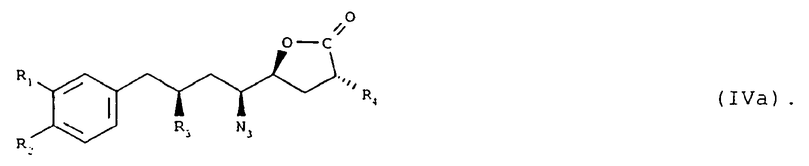

### Verfahrensstufe c)

Die Umsetzung der Verbindungen der Formel IVa mit einer Verbindung R₅NH₂ unter Oeffnung des Lactonringes wird zweckmässig in Gegenwart von Alkoholen oder Aminen durchgeführt, die aktivierte Carbonsäureester oder Carbonsäureamide zu bilden vermögen. Solche Verbindungen sind allgemein bekannt. Es kann sich um 2-Hydroxypyridin, N-Hydroxycarbonsäureamide und -imide, sowie Carbonsäureimide (N-Hydroxysuccinimid) handeln. Als Lösungsmittel verwendet man organische Lösungsmittel, vorteilhaft tertiäre Amine, zum Beispiel Trimethyl- oder Triethylamin. Die Reaktionstemperatur kann zum Beispiel etwa 40 °C bis 150 °C und vorzugsweise 50 °C bis 120 °C betragen.

Wenn man Verbindungen der Formel IVa einsetzt, erhält man bei der Umsetzung Verbindungen der Formel Va

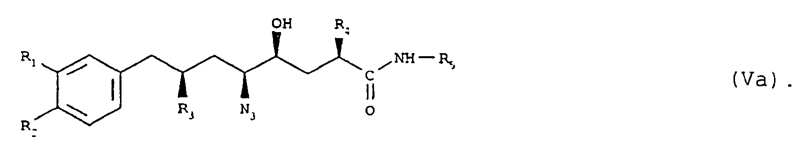

### Verfahrenstufe d)

Die Reduktion der Azidgruppe zur Amingruppe in den Verbindungen der Formeln Va erfolgt in an sich bekannter Weise (siehe Chemical Reviews, Vol. 88 (1988), Seiten 298 bis 317) zum Beispiel unter Verwendung von Metallhydriden oder zweckmässiger katalytisch mit Wasserstoff in Gegenwart von homogenen (Wilkinson-Katalysator) oder heterogenen Katalysatoren, zum Beispiel Raney-Nickel oder Edelmetallkatalysatoren wie Platin oder Palladium gegebenenfalls auf Trägermaterialien wie Kohlenstoff. Die Hydrierung kann auch gegebenenfalls katalytisch unter Transferbedingungen, zum Beispiel mit Ammoniumformiat als Wasserstoffdonor durchgeführt werden. Als Lösungsmittel verwendet man vorteilhaft organische Lösungsmittel. Die Reaktionstemperatur kann zum Beispiel etwa von 0 °C bis 200 °C und vorzugsweise 10 °C bis 100 °C betragen. Die Hydrierung kann bei Normaldruck oder erhöhtem Druck bis zum Beispiel 100 bar, bevorzugt bis zu 50 bar durchgeführt werden.

Wenn man Verbindungen der Formel Va einsetzt, erhält man Verbindungen der Formel Ia.

Die Verbindungen der Formel Ia können in an sich bekannter Weise durch Behandlung mit mono- oder mehrbasischen, anorganischen oder organischen Säuren in Additionssalze übergeführt werden. Bevorzugt sind Hemifumarate.

Die Halolaktonisierung der Verfahrensstufe a), die Azidierung der Verfahrensstufe b) und die Azidreduktion der Verfahrensstufe d) sind von P. Herold im Journal of Organic Chemistry, Vol. 54 (1989), Seiten 1178-1185 beschrieben.

Die Zwischenprodukte der Formeln IIa, und IIIa sind neu und stellen weitere Gegenstände der Erfindung dar.

Ein weiterer Gegenstand der Erfindung sind somit Verbindungen der Formel IIa 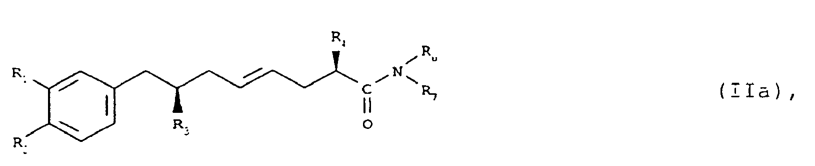 worin R₁, R₂, R₃, R₄, R₆ und R₇ die zuvor angegebenen Bedeutungen haben, einschliesslich der Bevorzugungen. Besonders bevorzugt bedeutet R₁ 1-Methoxy-prop-3-yloxy, steht R₂ für Methoxy, stellen R₃ und R₄ Isopropyl dar und bedeutet R₆ Methyl oder Ethyl, steht R₇ für Methyl, Ethyl oder Methoxy, oder bedeuten R₆ und R₇ zusammen Tetramethylen, Pentamethylen oder -CH (CH₂C₆H₅) CH₂-O-C(O)-.

Ein anderer Gegenstand der Erfindung sind Verbindungen der Formel IIIa 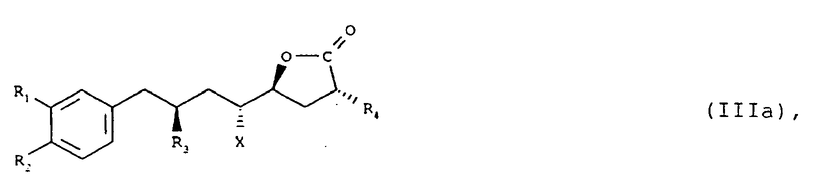 worin R₁, R₂, R₃, R₄ und X die zuvor angegebenen Bedeutungen haben, einschliesslich der Bevorzugungen. Besonders bevorzugt bedeutet R₁ 1-Methoxy-prop-3-yloxy, steht R₂ für Methoxy, stellen R₃ und R₄ Isopropyl dar und bedeutet X Cl, Br oder I.

Die Verbindungen der Formel IIa sind erhältlich, indem man eine Verbindung der Formel VI 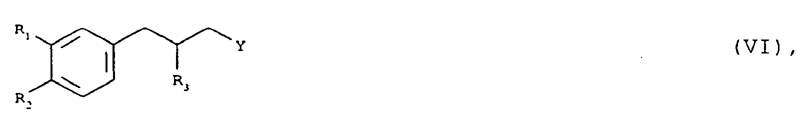 als Enantiomere, mit einer Verbindung der Formel VII, als Enantiomere, 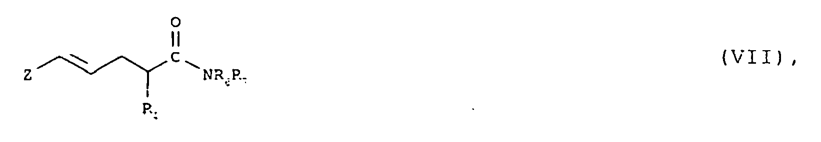 worin R₁ bis R₄, R₆ und R- die die zuvor angegebenen Bedeutungen haben, einschliesslich der Bevorzugungen, Y Cl, Br oder I und Z Cl, Br oder I bedeuten, in Gegenwart eines Alkali- oder Erdalkalimetalls umsetzt. Y und Z bedeuten bevorzugt Br und besonders Cl.

Die Kopplung von Grignardreagentien mit Alkenylhalogeniden in einem Ether wie zum Beispiel Tetrahydrofuran oder Dioxan als Lösungsmittel in Gegenwart von katalytischen Mengen eins löslichen Metallkomplexes, zum Beispiel Eisenkomplexes wie Eisenacetonylacetat, und in Gegenwart von mehr als äquimolaren Mengen eines die Metallkomplexe stabilisierenden Lösungsmittels, zum Beispiel N-Methylpyrrolidon, wird von G. Cahiez et al. in Synthesis (1998), Seiten 1199-1200 beschrieben. Die Reaktionstemperatur kann zum Beispiel -50 bis 80 °C, bevorzugt -20 bis 50 °C betragen. Katalytische Mengen kann zum Beispiel 0,1 bis 20 Gew.-% bedeuten, bezogen auf eine Verbindung der Formel VII. Zweckmässig wird die Umsetzung so geführt, dass man zunächst eine Verbindung der Formel VI in eine Grignardverbindung (zum Beispiel mit Magnesium) umwandelt und dann eine Lösung der Verbindung der Formel VII, Metallkomplex und N-Methylpyrrolidon zugibt, oder umgekehrt.

Es wurde überraschend gefunden, dass es vorteilhaft ist, wenn man nur katalytische Mengen eines die Metallkomplexe stabilisierenden Lösungsmittels, zum Beispiel N-Methylpyrrolidon verwendet. Katalytische Mengen kann zum Beispiel 1 bis 10 Mol-%, bevorzugt 1 bis 5 Mol-%, bezogen auf die Verbindungen der Formeln VI oder VII bedeuten.

Verbindungen der Formel VI in Form ihrer Racemate oder Enantiomeren sind bekannt oder nach analogen Verfahren herstellbar. Man kann zum Beispiel R₁R₂-Phenylaldehyd mit R₃-Diethoxy-phosphorylessigsäureester zu 2-R₃-3-(R₁R₂-Phenyl)-acrylsäureestern umsetzen, diese zu entsprechenden Propionsäureestern hydrieren, dann die Estergruppe verseifen und die Carbonsäure zum Alkohol reduzieren, und schliesslich die Hydroxylgruppe mit Halogen substituieren. Enantiomere sind über eine Racematspaltung der Carbonsäuren mit zum Beispiel Chinin, oder über eine enzymatische Racematspaltung der entsprechenden Carbonsäureester erhältlich. Einzelheiten sind in den Beispielen beschrieben. Eine mögliche asymmetrische Synthese von Verbindungen der Formel VI ist in der EP-A-0 678 503 beschrieben.

Die Verbindungen der Formel VII in Form ihrer Enantiomeren sind neu und ein weiterer Gegenstand der Erfindung. Ihre Herstellung kann durch Umsetzung von metalliertem Isovaleriansäureestern (zum Beispiel Lithium-Isovaleriansäureestern) mit 1,3-Tanshalogenpropen, anschliessender Halogenierung der gebildeten Carbonsäure zum Säurehalogenid und Umsetzung mit einem sekundären Amin erfolgen. Die Kopplung von Isovaleriansäure mit Trans-1,3-halogenpropen kann nach der von D. A. Evans in Asymmetric Synthesis, Vol. 3, 1984 (Academic Press Inc.), Seiten 2-110 beschriebenen Methode asymmetrisch durchgeführt werden. Weitere Einzelheiten sind in den Beispielen beschrieben. Enantiomere sind über eine Racematspaltung der Carbonsäuren mit zum Beispiel Cinchonidin, oder über eine enzymatisch Racematspaltung der entsprechenden Carbonsäureester erhältlich. Einzelheiten sind in den Beispielen beschrieben.

Ein weiterer Gegenstand der Erfindung sind Verbindungen der Formel VIIa 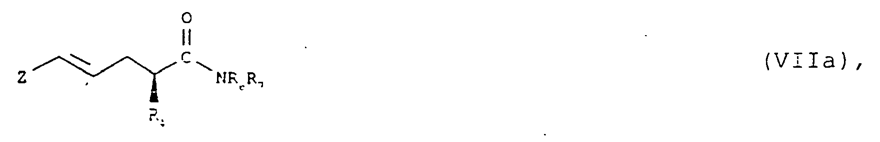 worin R₄, R₆ und R₇ die die zuvor angegebenen Bedeutungen haben, einschliesslich der Bevorzugungen, und Z für Cl, Br oder I und bevorzugt für Cl steht.

Mit der Wahl der Zwischenprodukte der Formel IIa können die an sich komplexen Verbindungen der Formel Ia in konvergenter und einfacher Weise hergestellt werden, was speziell auch für eine enantio- beziehungsweise diastereoselektive Synthese gilt. Die Gesamtausbeute über die Verfahrensstufen a) bis d) kann bis zu 25% und sogar 30% und mehr betragen, was eine industrielle Anwendung ermöglicht.

Die nachfolgenden Beispiele erläutert die Erfindung näher.

### A) Herstellung von Verbindungen der Formel VIa

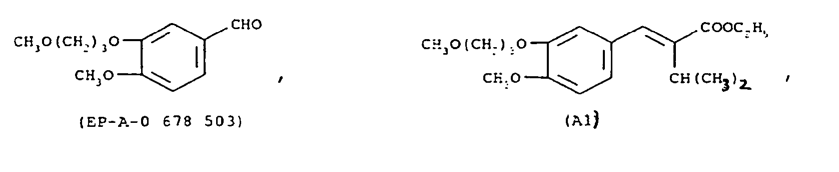

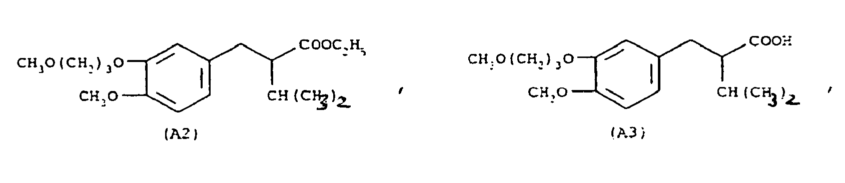

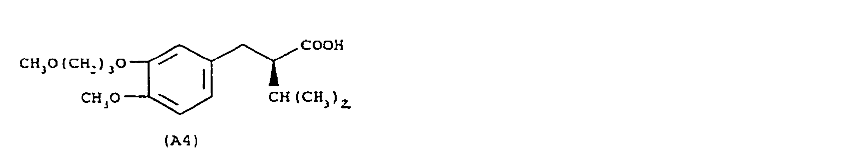

### Beispiel A1:

Zu einer Mischung von 18,8 g Kalium-tert.-butylat und 360 ml Tetrahydrofuran wird während 20 Minuten bei Raumtemperatur eine Lösung von 44,5 g 2-(Diethoxy-phosphoryl)-3-methyl-buttersäure-ethylester in 60 ml Tetrahydrofuran zugetropft. Nach weiteren 30 Minuten wird eine Lösung von 25,0 g 4-Methoxy-3-(3-methoxy-propoxy)-benzaldehyd (EP 0 678 503) in 100 ml Tetrahydrofuran zugetropft. Nach 14 Stunden wird das Reaktionsgemisch am Rotationsverdampfer (Rotavapor) eingeengt und der Rückstand zwischen Diethylether (3 x), Wasser (1x) und gesättigter wässriger Natriumchloridlösung extrahiert. Die vereinigten organischen Phasen werden mit Natriumsulfat getrocknet, filtriert und am Rotavapor eingedampft. Aus dem Rückstand wird mittels Flashchromatographie (SiO₂ 60F / Essigsäureethylester (Essigester) / Hexan 2:3) die Titelverbindung A1 als leicht gelbliches Oel erhalten (28,4 g, 75 %): ¹H-NMR (400 MHz, CDCl₃, δ): 1,15 - 1,42 (m, 9H), 2,12 (m, 2H), 2,70 - 3,30 (m, 1H), 3,40 (s, 3H), 3,60 (m, 2H), 3,85 - 4,35 (m, 7H), 6,40 - 7,50 (m, 4H) ppm.

### Beispiel A2:

26 g A1 werden in Gegenwart von 13 g RaNi in 500 ml Ethanol für 6 Stunden bei Raumtemperatur und Normaldruck hydriert. Das Reaktionsgemisch wird filtriert und am Rotavapor eingeengt. Aus dem Rückstand wird mittels Flaschromatographie (SiO₂ 60F/Essigester/Hexan 1:1) die Titelverbindung A2 als farbloses Oel erhalten (24,7 g, 94 %): ¹H-NMR (400Hz, CDCl₃, δ) : 1, 00 (d, 3H), 1,04 (d, 3H), 1,13 (t, 3H), 1,92 (m, 1H), 2,12 (m, 2H), 2, 47 (m, 1H), 2, 80 (m, 2H), 3,38 (s, 3H), 3,60 (t, 2H), 3,82 (s, 3H), 4,05 (m, 2H), 4,12 (t, 2H), 6,68 - 6, 80 (m, 3H) ppm.

### Beispiel A3:

Eine Mischung aus 27 g A2, 60 ml Ethanol und 60 ml 2N Natronlauge wird während 24 Stunden am Rückfluss gerührt. Das Reaktionsgemisch wird in 100 ml Wasser aufgenommen und mit Diethylether (2 x 200 ml) gewaschen. Die wässrige Phase wird mit 2N Salzsäure angesäuert und mit Essigester (4x) extrahiert. Die vereinigten organischen Phasen werden mit Natriumsulfat getrocknet, filtriert und am Rotavapor eingedampft. Aus dem Rückstand wird mittels Flashchromatographie (SiO₂ 60F / Dichlormethan / Methanol 20:1) die Titelverbindung A3 als leicht gelbliches Oel erhalten (23,1 g, 93 %) : ¹H-NMR (400 MHz, CDCl₃, δ): 0, 90 - 1,00 (m, 6H), 1, 80 (m, 1H), 1,95 (m, 1H), 1,95 (m, 1H), 2,35 (m, 1H), 2,70 (m, 2H), 3,30 (s, 3H), 3,50 (t, 2H), 3,75 (s, 3H), 3,98 (t, 2H), 6,60 - 6,90 (m, 3H), 11, 95 (s, 1H) ppm.

### Beispiel A4: Racematspaltung von Verbindung A3

5,0 g A3 werden in 15 ml Isopropanol und 210 ml Diisopropylether gelöst. Nach Zugabe von 2,61 g Chinin und 1,235 ml Triethylamin wird das Gemisch unter Rühren im Oelbad auf 50 °C erwärmt. Das Oelbad wird sodann entfernt und die klare Lösung mit 220 mg fein pulverisiertem Salz von A4 mit Chinin angeimpft. Man rührt während 2 Stunden bei Raumtemperatur und anschliessend noch 2 Stunden unter Eiskühlung. Der Niederschlag wird abfiltriert, mit 2 mal 50 ml eiskaltem Diisopropylether gewaschen und anschliessend im Vakuum bei 50 °C bis zur Gewichtskonstanz getrocknet. Man erhält 4, 22 g des angereicherten Salzes von A4 mit Chinin; Smp. 123 °C. Das so erhaltene Salz wird zwischen 250 ml Diethylether und 50 ml 1N HCl verteilt. Die wässrige Phase wird abgetrennt, die organische Phase mit gesättigter NaCl-Lösung gewaschen, mit MgSO₄ getrocknet und im Vakuum eingedampft. Man erhält 2, 22 g (44,4%) der angereicherten Verbindung A4.

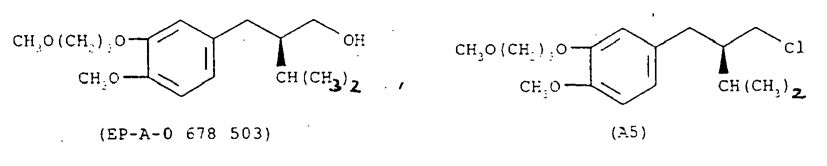

### Beispiel A5:

Eine gerührte Lösung von 174 g 2R-[4-Methoxy-3-(3-methoxy-propoxy)-benzyl]-3-methyl-butan-1-ol [EP 0678 503] und 1,3 Tetrachlorkohlenstoff wird auf 10° C gekühlt. Es werden 393 ml Trioctylphosphin zugetropft und die Reaktionslösung anschliessend 16 Stunden bei Raumtemperatur gerührt. Das Gemisch wird vollständig eingedampft und der Rückstand zwischen Methylenchlorid (3x) und wasser (1x) extrahiert. Die vereinigten organischen Phasen werden mit Magnesiumsulfat getrocknet , filtriert und eingedampft. Der Rückstand wird mittels Flashchromatographie (SiO₂ 60F / Essigester - Hexan 1:9) gereinigt und nach Kristallisation (Hexan bei -50° C) die Titelverbindung A5 als weisser Feststoff erhalten (152,3 g, 82 %) : Schmelzpunkt 51-52 °C; ¹H-NMR (400 MHz, CDCl₃, δ) : 1,0 (m, 6H), 1,71 (m, 1H), 1,93 (m, 1H), 2,12 (m, 2H), 2,53 (m, 1H), 2,77 (m, 1H), 3,39 (s, 3H), 3,40 - 3,55 (m, 2H), 3,71 (t, 2H), 3, 87 (s, 3H), 4,13 (t, 2H), 6,65 - 6,85 (m, 3H) ppm.

### B) Herstellung von Verbindungen der Formel VIIa

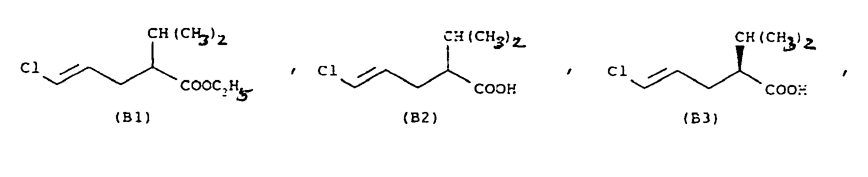

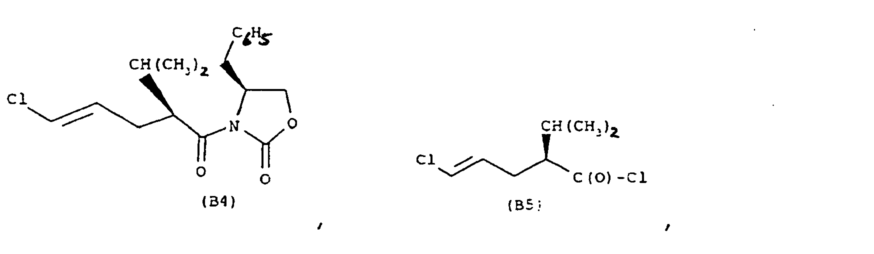

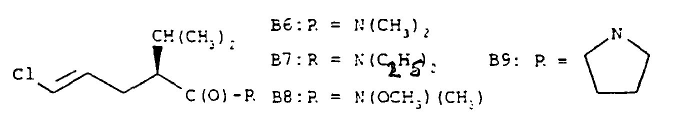

### Beispiel B1:

Eine gerührte Lösung von 24,9 ml Diisopropylamin und 240 ml Tetrahydrofuran wird auf minus 15 °C gekühlt und während 10 Minuten mit 100 ml 1,6 M n-Butyllithiumlösung (in Hexan) versetzt. Die Lösung wird 30 Minuten bei -15 °C nachgerührt und anschliessend wird während 30 Minuten eine Lösung von 24,1 ml Isovaleriansäureethylester in 80 ml Tetrahydrofuran zugetropft. Die Mischung wird weitere 5 Minuten bei -15° C gerührt und dann nacheinander mit 19,5 g trans-1,3-Dichlorpropen und 2,4 g Natriumiodid versetzt. Das Reaktionsgemisch wird noch 16 Stunden bei Raumtemperatur nachgerührt und anschliessend mit 500 ml 10%-iger wässriger Ammoniumchloridlösung versetzt. Die Mischung wird mit Diethylether extrahiert (3x) und die organischen Phasen nacheinander mit Wasser (1x), 0,1 M Natriumthiosulfatlösung (1x) und Sole (1x) gewaschen. Die vereinigten organische Phasen werden mit Natriumsulfat getrocknet und eingedampft. Aus dem Rückstand wird mittels Destillation die Titelverbindung B1 als farbloses Oel erhalten (24, 8 g, 76 %). ¹H-NMR (400 MHz, CDCl₃, δ) : 0,95 (m, 6H), 1,30 (t, 3H), 1,92 (m, 1H), 2,20 - 2,40 (m, 3H), 4,20 (m, 2H), 5,80 - 6,10 (m, 2H) ppm.

### Beispiel B2:

Eine Lösung von 150,2 g B1, 500 ml Ethanol und 500 ml 2N Natronlauge wird während 18 Stunden am Rückfluss gerührt. Aus dem Reaktionsgemisch wird das Ethanol abgedampft, die wässrige Lösung mit 1N Salzsäure angesäuert und mit Diethylether (3x) extrahiert. Die organischen Phasen werden mit Magnesiumsulfat getrocknet und eingedampft. Aus dem Rückstand wird mittels Flashchromatographie (SiO₂ 60F / Dichlormethan / Methanol 20:1) die Titelverbindung B2 als leicht oranges Oel erhalten (83, 7 g, 65 %). ¹H-NHR (400 MHz, CDCl₃, δ) : 1,03 (m, 6H), 1, 98 (m, 1H), 2,20 - 2,45 (m, 3H), 5,80 - 6,10 (m, 2H) ppm.

### Beispiel B3a: Racematspaltung von Verbindung B2

5,0 g B2, 5, 0 g Cinchonidin und 1,98 ml Triethylamin werden in 150 ml Tetrahydrofuran vorgelegt und während 15 Minuten unter Rückfluss gerührt. Das Oelbad wird entfernt und die klare Lösung mit einem Salz von B3 mit Cinchonidin angeimpft. Man rührt während 1 Stunde bei Raumtemperatur und anschliessend noch 1 Stunde unter Eiskühlung. Der Niederschlag wird abfiltriert, mit 2 mal 25 ml eiskaltem Aceton gewaschen und anschliessend im Vakuum bei 50 °C bis zur Gewichtskonstanz getrocknet. Man erhält 6,16 g (46,3%) des angereicherten Salzes von B3 mit Cinchonidin; Schmelzpunkt 149 °C. Nach zweimaliger Umkristallisation aus Aceton erhält man 4,20 g' (31,6%) des angereicherten Salzes von B3 mit Cinchonidin, Schmelzpunkt 155 °C. Das so erhaltene Salz wird zwischen 250 ml Diethylether und 50 ml 1N HCl verteilt. Die wässrige Phase wird abgetrennt, die organische Phase mit gesättigter NaCl-Lösung gewaschen, mit MgSO₄ getrocknet und im Vakuum eingedampft. Man erhält 1,58 g (31,6%) der angereicherten Verbindung B3 als farbloses Oel.

### Beispiel B3b: Asymmetrische Synthese von B3

Zu einer bei 0° C gerührten Lösung von 155 g B4, 1,3 Tetrahydrofuran und 0,44 1 Wasser werden während 15 Minuten 315 ml 30%-ige Wasserstoffperoxidlösung zugetropft. Das Reaktionsgemisch, wird mit 22,1 g Lithiumhydroxid versetzt, anschliessend das Kühlbad entfernt und dann 5 Stunden bei 0 - 20° C nachgerührt. Die Rekationsmischung wird wiederum auf 0° C gekühlt und während 30 Minuten eine Lösung von 350 g Natriumsulfit in 1,4 1 Wasser zugetropft. Durch Zugabe von Natriumydrogencarbonat wird auf pH 9,8 gestellt. Das Reaktionsgemisch wird klarfiltriert und aus dem Filtrat das Tetrahydrofuran abgedampft. Die erhaltene wässrige Lösung wird mit Methylenchlorid (3x 3 1) gewaschen. Die Wasserphase wird mit wässriger Salzsäure auf pH 3,.0 gestellt und dann mit Methylenchlorid (3x 21) extrahiert. Die organischen Phasen werden mit Magnesiumsulfat getrocknet und am Rotavapor eingedampft. Aus dem Rückstand wird mittels Destillation die Titelverbindung B3 als farbloses Oel erhalten. (142 g, 87 %). ¹H-NMR (400 MHz, CDCl₃, δ): 1,02 (m, 6H), 1,98 (m, 1H), 2,25 - 2,45 (m, 2H), 5,85 - 6,10 (m, 2H) ppm.

### Beispiel B4:

Eine Lösung von 290 g 4S-Benzyl-3-(3-methyl-butyryl)-oxazolidin-2-one in 0,58 1 Tetrahydrofuran wird auf -78 °C gekühlt und während 65 Minuten 1,14 1.1 M Lithiumhexamethyldisilazid (in Tetrahydrofuran) zugetropft. Das Gemisch wird noch 1 Stunde bei -78 °C nachgerührt und anschliessend mit der vorbereiteten Lösung von trans-1-Chloro-3-iod-propen in Tetrahydrofuran versetzt. Man lässt die Temperatur auf 0 °C steigen und rührt noch weitere 20 Stunden nach. Das Reaktionsgemisch wird mit 500 ml 10%-iger Ammoniumchloridlösung versetzt und mit Diethylether extrahiert (2x 1 1). Die organischen Phasen werden mit Wasser (1x 1 1) gewaschen, mit Natriumsulfat getrocknet und eingedampft. Aus dem Rückstand wird mittels Flashchromatographie (SiO 60F / Essigester/Hexan 5:1) die Titelverbindung B4 als leicht oranges Oel erhalten (582 g, 78 %). ¹H-NMR (400 MHz, CDCl₃, δ): 0, 85 (m, 6H), 2, 02 (m, 1H), 2,3 - 2,55 (m, 2H), 2,75 (m, 1H), 3,30 (m, 1H), 3,88 (m, 1H), 4,18 (m, 2H), 4,70 (m, 1H), 5,80 - 6,10 (m, 2H), 7,15 - 7,40 (m, 5H) ppm.
Herstellung von trans- 1-Chlor-3-iod-propen: Eine Lösung von 184.7 g trans-1,3-Dichlorpropen in 0,58 1 Tetrahydrofuran wird mit 266,1 g Natriumiodid versetzt und unter Lichtausschluss während 30 Minuten bei Raumtemperatur gerührt. Das Gemisch wird klarfiltriert und das Filtrat roh eingesetzt.

### Beispiel B5:

Eine Lösung von 4,54 g B3 in 25 ml Toluol wird bei Raumtemperatur mit 4,42 ml Oxalylchlorid versetzt. Das Reaktionsgemisch wird 15 Minuten bei Raumtemperatur gerührt und anschliessend während 1 Minute mit 0,052 ml N,N-Dimethylformamid versetzt. Das Reaktionsgemisch wird zum Rückfluss erwärmt und 1 Stunde gerührt. Die Reaktionslösung wird eingedampft und der Rückstand destilliert. Man erhält die Titelverbindung B5 als farbloses Oel: (4,43 g, 88 %). ¹H-NMR (400 MHz, CDCl₃, δ): 1,02 (d, 3H), 1,08 (d, 3H), 2,16 (m, 1H), 2,40 (m, 1H), 2,45 (m, 1H), 2,68 (m,1H), 5,80 - 6,10 (m, 2H) ppm.

### Beispiel B6:

Eine Lösung von 1,53 g Dimethylamin, 3,66 ml Pyridin und 25 ml Dichlormethan wird auf 0 °C gekühlt und anschliessend 4,42 g B5 in 25 ml Dichlormethan bei 0 bis -10° C zugetropft. Das Reaktionsgemisch wird noch 2 Stunden bei 0 °C weitergerührt und' anschliessend am Rotavapor eingedampft. Der Rückstand wird zwischen Diethylether (2x) und 2N Salzsäure (3x), gesättigter Natriumhydrogencarbonatlösung (1x) und gesättigter Kochsalzlösung verteilt. Die organischen Phasen werden vereinigt, über Natriumsulfat getrocknet und eingeengt. Der Rückstand wird destilliert und die Titelverbindung B6 als farbloses oel erhalten: (4,13 g, 89 %). [α]²⁵ d⁻ 7,3 (c 1, Chloroform). ¹H-NMR (400 MHz, CDCl₃, δ): 0,90 (d, 3H), 0,95 (α, 3H) , 1,92 (m, 1H), 2,20 - 2,30 (m, 1H), 2,35 - 2,50 (m, 2H), 2,98 (s, 3H., 3,04 (s, 3H), 5,80 - 6,10 (m, 2H) ppm.

Analog Beispiel B6 werden aus dem Säurechlorid B5 und den entsprechenden Aminen, die Derivate B7, B8 und B9 hergestellt.

### C) Herstellung von Verbindungen der Formel IIa

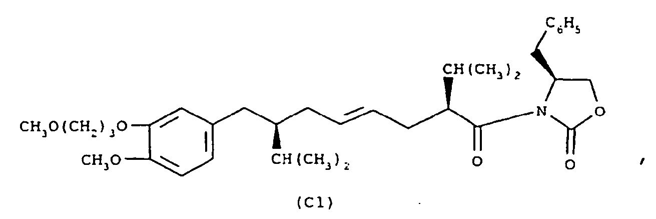

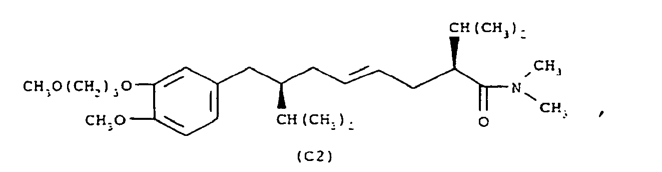

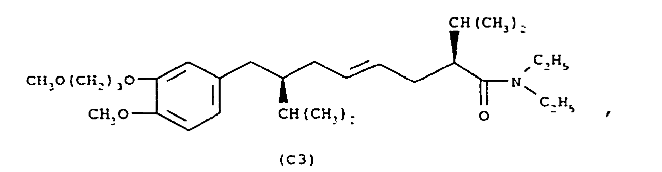

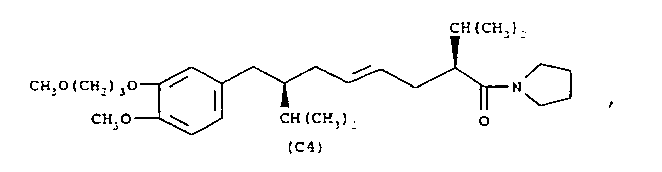

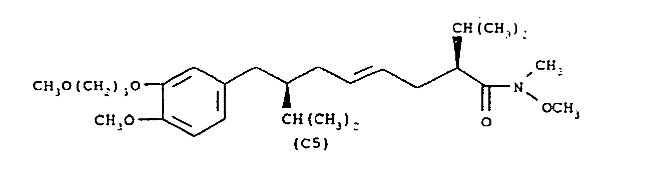

### Beispiel C1:

Eine Mischung von 4,28 g Magnesium-Pulver und 50 ml Tetrahydrofuran wird auf 60 °C erwärmt und dann während 2 Minuten mit 0,30 ml 1,2-Dibromethan versetzt (sichtbare exotherme Reaktion). Eine Lösung aus 13,85 g A5, 1,6 ml 1,2-Dibromethan und 130 ml Tetrahydrofuran wird während 15 Minuten bei 60 - 64° C zugetropft. Das Gemisch wird noch 30 Minuten am Rückfluss gerührt und anschliessend auf Raumtemperatur abgekühlt. Das Reaktionsgemisch wird unter Argon klarfiltriert und das Filtrat in einem zweiten Reaktionsgefäss auf -2 °C gekühlt. Eine Lösung von 13,43 B4, 0.71 g Eisen (III) - acetylacetonat, 0,192 ml N-Methylpyrrolidon und 80 ml Tetrahydrofuran wird bei -2 bis 5 °C während 2 Minuten zugetropft. Das Reaktionsgemisch wird noch 10 Minuten bei 0 °C nachgerührt und anschliessend mit 140 ml 2N Salzsäure versetzt. Man extrahiert mit Diethylether (2x) und wäscht die organischen Phasen nacheinander mit Wasser (1x) und gesättigter wässriger Natriumchloridlösung (1x) . Die vereinigten organischen Phasen werden mit Natriumsulfat getrocknet, filtriert und am Rotavapor eingedampft. Aus dem Rückstand wird mittels Flashchromatographie (SiO. 60F / Diethylether /Hexan 1:1) die Titelverbindung C1 als farbloses Oel erhalten. (13,7 g, 59 %): TLC R- = 0,11 (Diethylether / Hexan 1:1); ¹H-NMR (500 MHz, CDCl₃, δ): 0,81 -0,90 (m, 6H), 0,97 (d, 6H), 1,52 (m, 1H), 1,70 (m, 1H), 1,84 - 1,91 (m, 1H), 1,93 - 2,03 (m, 2H) , 2,08 (m, 2H), 2,31 - 2,39 (m, 2), 2, 41 - 2, 59 (m, 3H), 3,29 (dd, 1H), 3,36 (s, 3H), 3,57 (t, 2H), 3,80 (s, 3H), 3,78 - 3,85 (m, 1H), 4,03 - 4,16 (m, 4H), 4,67 (m, 1H), 5,37 - 5,5. (m, 2H), 6,63 (dd, 1H), 6,67 (d, 1H), 6,70 (d, 1H), 7,14 - 7,34 (m, 5H) ppm.

### Beispiel C2:

Eine Mischung von 10,7 g Magnesium-Pulver und 120 ml Tetrahydrofuran wird auf 60 °C erwärmt und dann während 2 Minuten mit 0,74 ml 1,2-Dibromethan versetze (sichtbare exotherme Reaktion). Eine Lösung aus 34,6 g A5, 4,0 ml 1,2-Dibromethan und 320 ml Tetrahydrofuran wird während 15 Minuten bei 62 - 64°C zugetropft. Das Gemisch wird noch 30 Minuten am Rückfluss gerührt und anschliessend auf Raumtemperatur abgekühlt. Das Reaktionsgemisch wird unter Argon klarfiltriert und die erhaltene Grignardlösung während 10 Minuten zu einer Lösung von 20,4 g B4, 0,240 ml N-Methylpyrrolidon, 0,88 g Eisen (III)-acetylacetonat in 200 ml Tetrahydrofuran, bei - 5 bis 0°C, getropft. Das Reaktionsgemisch wird noch 15 Minuten bei 0° C nachgerührt und anschliessend bei 0 bis 10°C mit 320 ml 2N Salzsäure versetzt. Man extrahiert nun mit Diethylether (3x 500 ml) und wäscht die organischen Phasen nacheinander mit Wasser (1x 400 ml) und gesättigter wässriger Natriumchloridlösung (1x 400 ml). Die vereinigen organischen Phasen werden mit Natriumsulfat getrocknet, filtriert und am Rotavapor eingedampft. Aus dem Rückstand wird mittels Flashchromatographie (SiO₂ 60F / Diethylether / Hexan 2:1) die Titelverbindung C2 als leicht gelbliches oel erhalten (36,2 g, 81%): TLC Rᵣ = 0, 09 (Diethylether / Hexan 2:1); ¹H-NMR (500 MHz, CDCl₃, δ) 0,82 - 0,99 (m, 12H), 1,49 (m, 1H), 1,69 (m, 1H), 1,78 - 1,98 (m, 3H), 2,10 (m, 2H), 2,17 - 2,41 (m, 5H), 2,92 (s, 3H), 3,0 (s, 3H), 3,37 (s, 3H), 3,56 (t, 2H), 3,84 (s, 3H), 4,10 (t, 2H), 5,26 - 5,34 (m, 1H), 5,36 - 5,44 (m, 1H); 6,64 (m, 2H), 6,78 (d, 1H) ppm.

Analog Beispiel C2 werden durch Umsetzen von der Verbindung A5 mit den Verbindungen B7, B8 und B9 die Verbindungen C3, C4 und C5 hergestellt.

### D) Halolaktonisierung

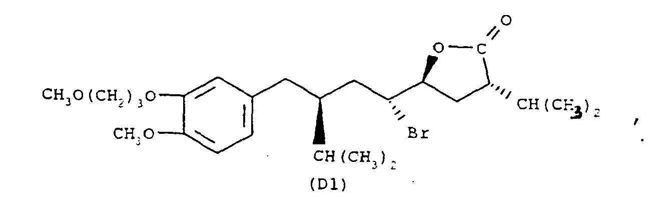

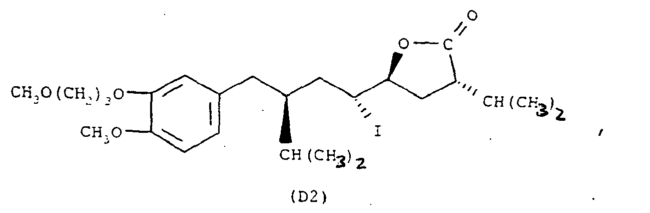

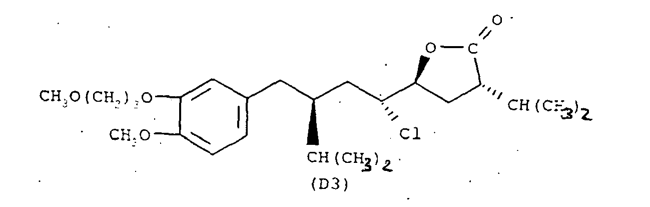

### Beispiel D1:

Eine Lösung von 34,2 g C2 und 385 ml Tetrahydrofuran wird mit 3,85 ml Wasser versetzt und unter Rühren auf 0 °C gekühlt. Dann wird abwechslungsweise 10 mal 1,03 ml 42,5 %-ige o-Phösphörsäure und 10 mal 1,5 g N-Bromsuccinimic im Abstand von jeweils 3 Minuten zugegeben. Das Reaktionsgemisch wird noch 90 Minuten bei 0° C nachgerührt und anschliessend während 10 Minuten in 600 ml auf 0 °C gekühlte Natriumhydrogensulfitlösung eingetragen. Die Mischung wird noch 15 Minuten bei 0 °C gerührt und anschliessend mit Diethylether (1x 11 und 2x 0.5, 1) extrahiert. Die organischen Phasen werden nacheinander mit 1N Salzsäure (1x 0.6 1), Wasser (1x 0.6 1), gesättigter wässriger Natriumhydrogencarbonatlösung (1x 0.6 1) und Sole (1x 0.6 1) gewaschen, mit Natriumsulfat getrocknet und am Rotavapor eingeengt. Aus dem Rückstand wird durch Kristallisation (Diisopropylether-Hexan 1:2 bei -25 °C) die Titelverbindung D1 als weisses Kristallisat erhalten (27,5 g, 72 %): Schmelzpunkt 48 - 49 °C; TLC R- = 0,09 (Diethylether / Hexan 2:1); [α]²⁵_{D} = 44,2 (c 1, Chloroform); ¹H-NMR (500 MHz, CDCl₃, δ): 0,85 - 1,07 (m, 12H), 1,57 - 1,65 (m, 1H), 1,79 - 2,00 (m, 3H) , 2, 07 - 2,27 (m, 6H), 2,62 (m, 1H) , 2, 75 (dd, 1H), 3,37 (s, 3H), 3,59 (t, 2H), 3,86 (s, 3H), 4,02 (m, 1H) , 4,12 (t, 2H), 4, 35 (m, 1H), 6, 72 (dd, 1H), 6,75 (d, 1H), 6,81 (d, 1H) ppm. D1 kann in analoger Weise auch aus C3, C4 und C5 hergestellt werden.

### Beispiel D2:

Eine Mischung von 1,72 g C1, 36 ml Dichlormethan und 12 ml Wasser wird mit 0,56 g N-Bromsuccinimid versetzt und während 24 Stunden bei 35 °C gerührt. Die organische Phase wird abgetrennt, mit 0,1 N Natriumthiosulfatlösung und Sole gewaschen, mit Natriumsulfat getrocknet und am Rotavapor eingeengt. Aus dem Rückstand wird nach Flashchromatographie (SiO₂ 60F / Dimethylether / Hexan 1:1) und Kristallisation (Diisopropylether/Hexan 1:2 bei -25 °C) die Titelverbindung D1 als weisses Kristallisat erhalten (0,61 g, 41%).

In ähnlicher Weise werden aus C2 die Derivate D2 und D3 erhalten:
- D2:: ¹H-NMR (500 MHz, CDCl₃, δ): 0,85 - 1,07 (m, 12H), 1,57 - 1,66 (m, 1H), 1,6 9 - 1,9 3 (m, 3H), 2,04 - 2,26 (m, 6H), 2,62 (m, 1H), 2,77 (dd, 1H), 3, 37 (s, 3H), 3,59 (t, 2H), 3,86 (s, 3H), 4,20 (m, 1H), 4,12 (t, 2H), 4,20 (m, 1H), 6,70 (dd, 1H), 6,73 (d, 1H), 6,80 (d, 1H) ppm.
- D3:: ¹H-NMR (500 MHz, CDCl₃, δ): 0,85 - 1,07 (m, 12H) , 1,47 - 1,55 (m, 1H), 1,75 - 1,97 (m, 3H), 2,C7 - 2,27 (m, 6H), 2,62 (m, 1H), 2,74 (dd, 1H), 3,37 (s, 3H), 3,59 (t, 2H), 3,86 (s, 3H), 3,98 (m, 1H), 4,12 (t, 2H), 4,35 (m, 1H), 6,72 (dd, 1H), 6,75 (d, 1H), 6,81 (d, 1H) ppm.

### E) Azidierung

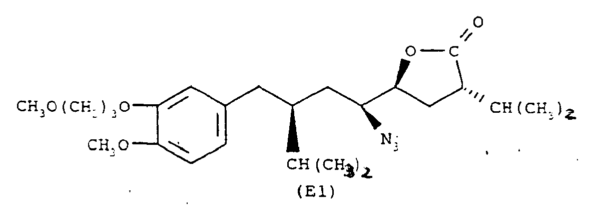

### Beispiel E1:

Eine Mischung von 63,1 g D1, 39,0 g Natriumazid und 450 ml Tripropylenglykol und 150 ml Wasser wird während 41 Stunden bei 80 °C gerührt. Das Reaktionsgemisch wird auf Raumtemperatur gekühlt und mit 30 ml Dimethylamino-1-propylamin versetzt und anschliessend während 3 Stunden bei Raumtemperatur nachgerührt. Das Reaktionsgemisch wird auf 750 ml Wasser gegossen und mit tert.-Butylmethylether (3x 750 ml) extrahiert. Die organischen Phasen werden nacheinander mit 750 ml 0.5 N HCl, 750 ml NaHCO₃, (5%-ig in Wasser), Wasser (3x 750 ml) und 750 ml Sole gewaschen. Die organischen Phasen werden mit 150 g Natriumsulfat getrocknet, filtriert und am Rotationsverdampfer eingeengt. Aus dem Rückstand wird mittels Flashchromatographie (SiO 60F / Essigester / Hexan 1:3) die Titelverbindung E1 als leicht farbloses Oel erhalten (32.1 g, 58 %) : 0,89 - 0,98 (m, 9H), 1,03 (d, 3H), 1,33 -1,41 (m, 1H), 1,65 - 1,85 (m, 3H), 1,91 -2,01 (m, 1H), 2,02 - 2,20 (m, 4H), 2,42 - 2,49 (m, 1H), 2,55 - 2,65 (m, 2H), 2,92 - 2, 97 (m, 1H), 3,37 (s, 3H), 3,59 (t, 2H), 3,85 (s, 3H), 4,12 (t, 2H) , 4,27 (m, 1H), 6,72 (dd, 1H), 6,75 (d, 1H), 6,81 (d, 1H) ppm.

### F) Amidierung des Lactons

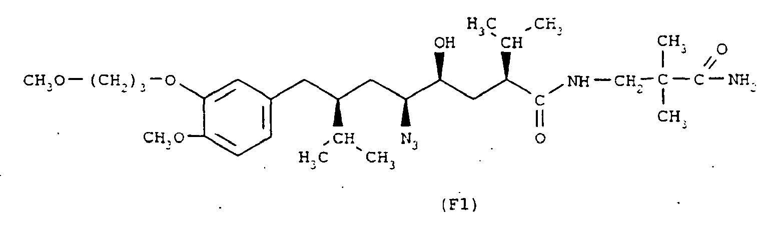

### Beispiel F1:

Eine Mischung von 59,1 g (HPLC-Gehalt: 93,8 %-ig) E1, 41, 82 g 3-Amino-2,2-dimethyl-propionamide, 2,28 g 2-Hydroxypyridin in 59,1 ml Triethylamin wird während 16 Stunden bei 90 °C gerührt. Dann werden 33 ml Triethylamin während 0,5 Stunden abdestilliert und der Rückstand weitere 8,5 Stunden bei 90°C gerührt. Das abgekühlte Reaktionsgemisch wird zwischen Essigester (3x 500 ml), gesättigter wässriger Natriumhydrogencarbonatlösung (1x 500 ml) und gesättigter Natriumchloridlösung (1x 500 ml) extrahiert. Die vereinigten organischen Phasen werden mit 100g Natriumsulfat getrocknet, filtriert und am Rotationsverdampfer eingeengt. Der Rückstand wird getrocknet und die rohe Titelverbindung F1 als Oel erhalten (78,4g, quantitativ) (HPLC-Gehalt: 88,5 -ig) : TLC Rₜ = 0,13 (Essigester / Hexan 4:1); Chromatographierte Probe: TLC Rₜ = 0,13 (Essigester / Hexan 4:1) ;¹H-NMR (500. MHz, CDCl₃, δ): 0,85 - 0,96 (m, 12H), 1,23 (s, 6H), 1,30 - 1,40 (m, 1H) 1,53 - 1,80 (m, 5H), 1,82 - 1.93 (m, 1H), 2,06 - 2,14 (m, 3H), 2,45 - 2, 57 (m, 2H), 2,87 - 2,92 (m, 1H), 3,13 (d, 1H), 3,32 - 3,52 (m, 3H), 3,36 (s, 3H), 3,59 (t, 2H), 3,84 (s, 3H), 4,12 (t, 2H), 5,51 (bs, 1H), 6,01 (bs, 1H), 6,43 (t, 1H), 6,72 (dd, 1H), 6,75 (d, 1H), 6,81 (d, 1H) ppm.

### G) Hydrierung der Azidgruppe

### Beispiel G1:

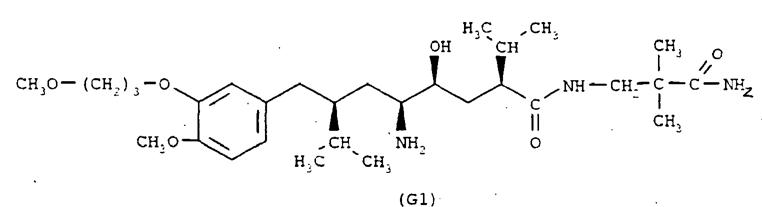

78, 4 g (HPLC-Gehalt: 88,5 %-ig) F1 (roh) werden in Gegenwart von 3,92 g Pd/C 5% und 7,2 ml Ethanolamin in 700 ml tert.-Butylmethylether für 3 Stunden bei Raumtemperatur und 3,0 bar hydriert. Das Reaktionsgemisch wird filtriert und der Katalysator mit 300 ml tert.-Butylmerhylether gewaschen. Das Filtrat wird nacheinander mit 400 ml 2N NaOH und 400 ml Sole gewaschen. Die wässrigen Phasen werden mit tert.-Butylmethylether (2 x 400 ml) nachextrahiert. Die vereinigten organischen Phasen werden mit 100 g Natriumsulfat getrocknet und eingeengt. Der Rückstand wird mit 7,31 g fumarsäure versetzt und in 200 ml Ethanol bei 35° C gelöst und klarfiltriert. Das Filtrat wird auf ein Totalgewicht von 104 g eingeengt und bei 35° C in 1,7 1 Acetonitril gelöst. Die erhaltene Lösung wird mit 10 mg der Titelverbindung (Hemifumarat) angeimpft und. 17 Stunden bei Raumtemperatur gerührt. Die Suspension wird auf 0° C gekühlt und nach 2 Stunden abgenutscht. Der Rückstand wird mit Acetonitril (3 x 200 ml) gewaschen und anschliessend bei 35° C inm Vakuum getrocknet. Man erhält die Titelverbindung (Hemifumarat) als weisse Kristalle (59,5 g, 81 % bezogen auf E1): ¹H NMR (360 MHz, DMSO-d₆) ; δ 0,7 - 0,9 (m, 12H), 1,04 (s, 6H), 1,27 (m, 3H), 1,4 - 1,8 (m, 4H), 1,94 (m, 2H), 2,23 (m, 1H), 2,35 (dd, J = 8,4, 8,0 Hz, 1H), 2,45 (m, 1H), 3,08 (m, 2H), 3,2 *-* 3,5 (m, 2H), 3,24 (s, 3H), 3,47 (t, J = 6,4 Hz, 2H), 3,74 (s, 3H), 3,97 (t, J = 6,4 Hz, 2H), 6,37 (s, 1H), 6,68 (dd, J = 8, 0, 2, 0 Hz, 1H), 6,77 (d, J = 6 Hz, 1H), 6,80 (bs, 1H), 6,83 (d, J = 8 Hz, 1H), 7,13 (bs, 1H 7,49 (t, J = 6 Hz, 1H).

## Patentansprüche

1. Verfahren zur Herstellung von Verbindungen der Formel Ia, 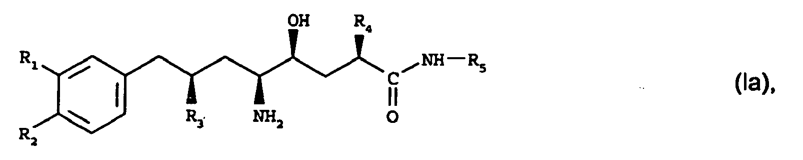 worin
R₁ und R₂ unabhängig voneinander H, C₁-C₆-Alkyl, C₁-C₆-Halogenalkyl, C₁-C₆-Alkoxy, C₁-C₆-Alkoxy-C₁-C₆-Alkyl, oder C₁-C₆-Alkoxy-C₁-C₆-Alkyloxy darstellen, R₃ C₁-C₆-Alkyl bedeutet, R₄ für C₁-C₆-Alkyl steht, und R₅ C₁-C₆-Alkyl, C₁-C₆-Hydroxyalkyl, C₁-C₆-Alkoxy-C₁-C₆-alkyl, C₁-C₆-Alkanoyloxy-C₁-C₆-alkyl, C₁-C₈-Aminoalkyl, C₁-C₆-Alkylamino-C₁-C₆-alkyl, C₁-C₆-Dialkyl-amino-C₁-C₆-alkyl, C₁-C₈-Alkanoylamido-C₁-C₆-alkyl, HO(O)C-C₁-C₆-alkyl, C₁-C₆-Alkylo-(O)C-C₁-C₆-alkyl, H₂N-C(O)-C₁-C₆-alkyl, C₁-C₆-Alkyl-HN-C(O)-C₁-C₆-alkyl oder (C₁-C₆-Alkyl)₂N-C(O)-C₁-C₆-alkyl darstellt, **dadurch gekennzeichnet, dass** man
a) eine Verbindung der Formel IIa 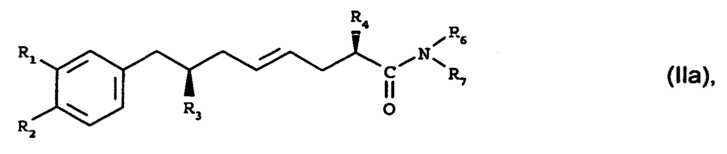 worin
R₈ C₁-C₆-Alkyl darstellt, R₇ C₁-C₆-Alkyl oder C₁-C₆-Alkoxy bedeutet, oder R₆ und R₇ zusammen gegebenenfalls mit C₁-C₄-Alkyl, Phenyl oder Benzyl substituiertes Tetramethylen, Pentamethylen, 3-Oxa-1,5-Pentylen oder -CH2CH₂O-C(O)- sind, mit einem Halogenierungsmittel in Gegenwart von Wasser und gegebenenfalls einer Säure zu einer Verbindung der Formel IIIa umsetzt, 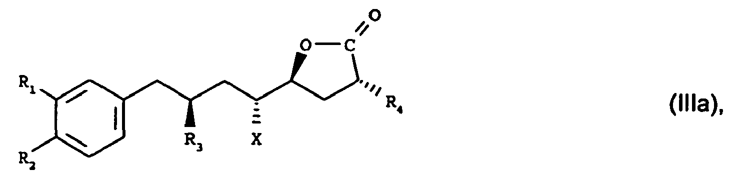 worin X für Cl, Br oder I steht,
b) die Verbindung der Formel IIIa mit einem Azidierungsmittel zu einer Verbindung der Formel IVa umsetzt, 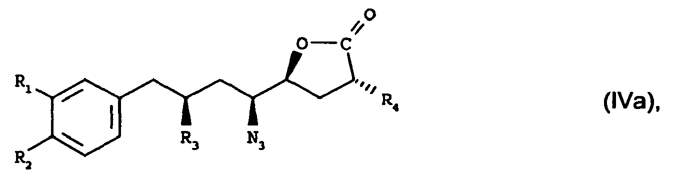
c) danach die Verbindung der Formel IVa mit einem Amin der Formel R₅-NH₂ zu einer Verbindung der Formel Va umsetzt, 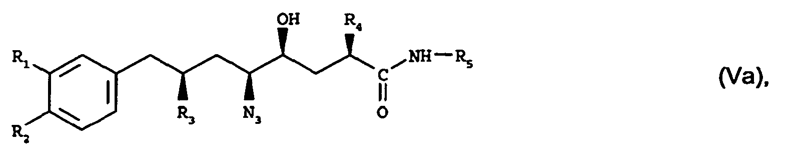 und
d) zur Herstellung der Verbindung der Formel Ia die Azidgruppe der Verbindung der Formel Va zur Amingruppe reduziert, und dann die Verbindungen der Formel Ia isoliert, gegebenenfalls unter Zugabe einer salzbildenden Säure.

2. Verfahren gemäss Anspruch 1, **dadurch gekennzeichnet, dass** R₁ C₁-C₄-Alkoxy oder C₁-C₄-Alkoxy-C₁-C₄-Alkyloxy bedeutet, R₂ C₁-C₄-Alkoxy darstellt, R₃ C₁-C₄-Alkyl ist, R₄ C₁-C₄-Alkyl bedeutet und R₅ für gegebenenfalls N-Mono- oder N-Di-C₁-C₄-Alkyl substituiertes H₂NC(O)-C₁-C₆-Alkyl steht.

3. Verfahren gemäss Anspruch 2, **dadurch gekennzeichnet, dass** R₁ 1-Methoxyprop-3-yloxy und R₂ Methoxy bedeuten.

4. Verfahren gemäss Anspruch 2, **dadurch gekennzeichnet, dass** R₃ und R₄ je Isopropyl darstellen.

5. Verfahren gemäss Anspruch 2, **dadurch gekennzeichnet, dass** R₅ für H₂NC(O)-C₁-C₆-Alkyl steht.

6. Verfahren gemäss Anspruch 1, **dadurch gekennzeichnet, dass** R₁ Methoxy-C₁C₄-Alkyloxy bedeutet, R₂ Methoxy oder Ethoxy darstellt, R₃ C₂₋C₄-Alkyl ist, R₄ C₂-C₄-Alkyl bedeutet und R₅ für H₂NC(O)-C₁-C₆-Alkyl steht.

7. Verfahren gemäss Anspruch 1, **dadurch gekennzeichnet, dass** R₁ 3-Methoxy-prop-3-yloxy bedeutet, R₂ Methoxy darstellt, R₃ und R₄ je für 1-Methyleth-1-yl stehen, und R₅ H₂NC(O)-[C(CH₃)₂] -CH₂- darstellt.

8. Verbindungen der Formel IIa 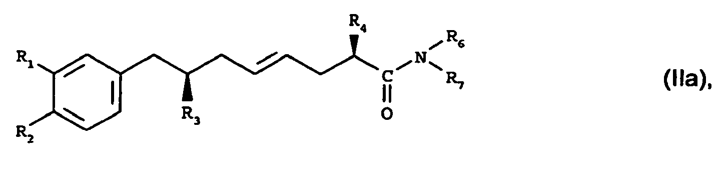 worin R₁, R₂, R₃, R₄, R₆ und R₇ die in Anspruch 1 angegebenen Bedeutungen haben.

9. Verbindungen gemäss Anspruch 8, **dadurch gekennzeichnet, dass** R₁ 1-Methoxy-prop-3-yloxy bedeutet, R₂ für Methoxy steht, R₃ und R₄ Isopropyl darstellen, und R₈ Methyl oder Ethyl bedeutet, R₇ für Methyl, Ethyl oder Methoxy steht, oder R₆ und R₇ zusammen Tetrametylen, Pentamethylen oder -CH(CH₂C₆H₅)CH₂-O-C(O)- bedeuten.

10. Verbindungen der Formel IIIa 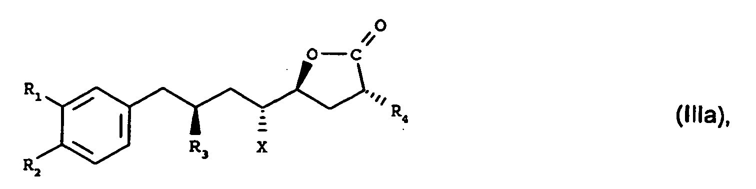 worin R₁, R₂, R₃, R₄ und X die in Anspruch 1 angegebenen Bedeutungen haben.

11. Verbindungen gemäss Anspruch 10, **dadurch gekennzeichnet, dass** R₁ 1-Methoxy-prop-3-yloxy bedeutet, R₂ für Methoxy steht, R₃ und R₄ Isopropyl darstellen und X Cl, Br oder I bedeutet.

12. Verbindungen der Formel Vlla 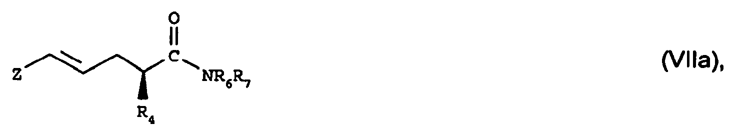 worin R₄, R₆ und R₇ die in Anspruch 1 angegebenen Bedeutungen haben, und Z für Cl, Br oder I steht.

13. Verbindungen gemäss Anspruch 12, **dadurch gekennzeichnet, dass** R₄ 1-Methylethyl bedeutet, Z für Cl steht, und R₆ Methyl oder Ethyl bedeutet, R₇ für Methyl, Ethyl oder Methoxy steht, oder % und R₇ zusammen Tetrametylen, Pentamethylen oder -CH(CH₂C₆H₅)CH₂O-C(O)- bedeuten.

14. Verbindungen der Formel IVa 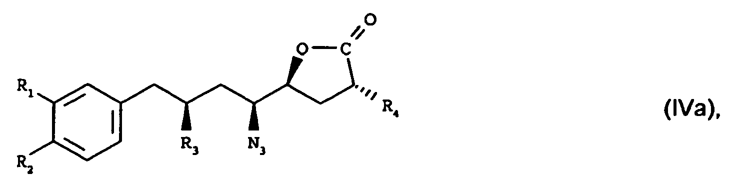 worin R_{1,} R₂, R₃ und R₄ die in Anspruch 1 angegebenen Bedeutungen haben.

## Claims

1. Process for preparation of compounds of formula la, 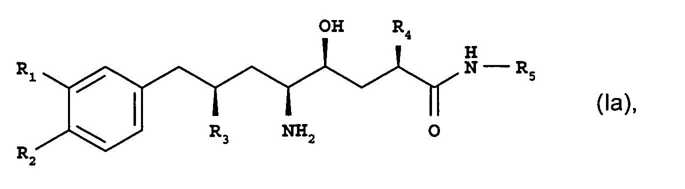 wherein
R₁ and R₂ are independently of one another H, C₁-C₆alkyl, C₁-C₆halogenalkyl, C₁-C₆alkoxy, C₁-C₆alkoxy-C₁-C₆alkyl, or C₁-C₆alkoxy-C₁-C₆alkyloxy, R₃ is C₁-C₆alkyl, R₄ is C₁-C₆alkyl, and R₅ is C₁-C₆alkyl, C₁-C₆hydroxyalkyl, C₁-C₆alkoxy-C₁-C₆-alkyl, C₁-C₆alkanoyloxy-C₁-C₆alkyl, C₁-C₆aminoalkyl, C₁-C₆alkylamino-C₁-C₆-alkyl, C₁-C₆-dialkylamino-C₁-C₆-alkyl, C₁-C₆-alkanoylamido-C₁-C₆-alkyl, HO(O)C-C₁-C₆-alkyl, C₁-C₆alkyl-O-(O)C-C₁-C₆alkyl, H₂N-C(O)-C₁-C₆alkyl, C₁-C₆alkyl-HN-C(O)-C₁-C₆alkyl or (C₁-C₆alkyl)₂N-C(O)-C₁-C₆-alkyl, comprising
a) the reaction of a compound of formula IIa 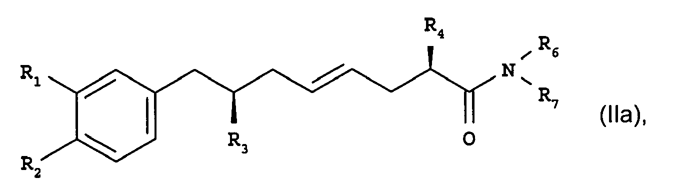 wherein
R₆ is C₁-C₆alkyl, R₇ is C₁-C₆alkyl or C₁-C₆alkoxy, or R₆ and R₇ together are tetramethylene, pentamethylene, 3-oxa-1,5-pentylene or -CH₂CH₂O-C(O)- substituted if necessary with C₁-C₄alkyl, phenyl or benzyl, with a halogenation agent in the presence of water, and if necessary, an acid to form a compound of formula IIIa, 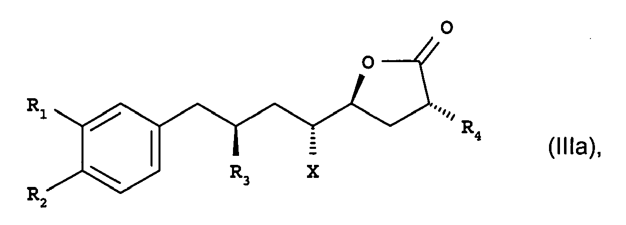 wherein X is Cl, Br or I,
b) reaction of the compound of formula IIIa with an azidation agent to form a compound of formula lVa, 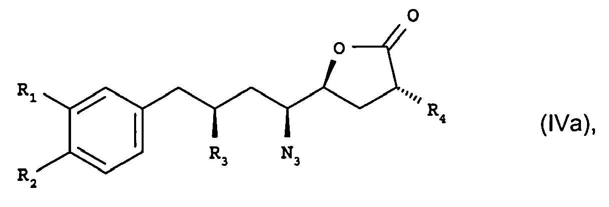
c) thereafter reaction of the compound of formula IVa with an amine of formula R₅-NH₂ to form a compound of formula Va, 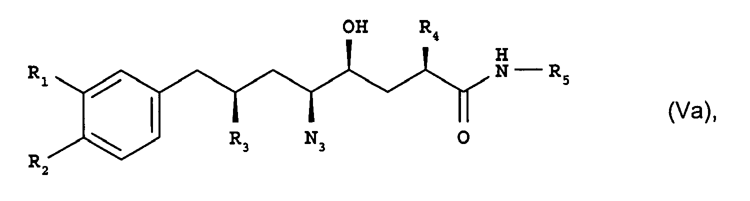 and
d) for preparation of a compound of formula la, reduction of the azide group of the compound of formula Va to form the amine group and then isolation of the compounds of formula la, if necessary with the addition of a salt-forming acid.

2. A process according to claim 1 comprising an embodiment wherein R₁ is C₁-C₄alkoxy or C₁-C₄alkoxy-C₁-C₄alkyloxy, R₂ is C₁-C₄alkoxy, R₃ is C₁-C₄alkyl, R₄ is C₁-C₄alkyl and R₅ is H₂NC(O)-C₁-C₆alkyl which if necessary is N-monosubstituted or N-di-C₁-C₄alkyl substituted.

3. A process according to claim 2 comprising an embodiment wherein R₁ is 1-methoxyprop-3-yloxy and R₂ is methoxy.

4. A process according to claim 2 comprising an embodiment wherein R₃ and R₄ are in each case isopropyl.

5. A process according to claim 2 comprising an embodiment wherein R₅ is H₂NC(O)-C₁-C₆alkyl.

6. A process according to claim 1 comprising an embodiment wherein R₁ is methoxy-C₂-C₄alkyloxy, R₂ is methoxy or ethoxy, R₃ is C₂-C₄alkyl, R₄ is C₂-C₄alkyl and R₅ is H₂NC(O)-C₁-C₆alkyl.

7. A process according to claim 1 comprising an embodiment wherein R₁ is 3-methoxy-prop-3-yloxy, R₂ is methoxy, R₃ and R4 are 1-methyleth-1-yl, and R₅ is H₂NC(O)-[C(CH₃)₂](-CH₂-.

8. Compounds of formula IIa 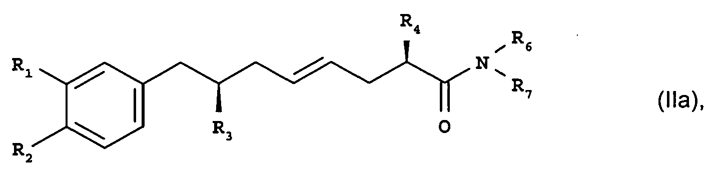 wherein R₁, R₂, R₃, R₄, R₆ and R₇ are as defined in claim 1.

9. Compounds according to claim 8, comprising an embodiment wherein R₁ is 1-methoxyprop-3-yloxy, R₂ is methoxy, R₃ and R₄ are isopropyl and R₆ is methyl or ethyl, R₇ is methyl, ethyl or methoxy, or R₆ and R₇ together are tetramethylene, pentamethylene or - CH(CH₂C₆H₅)CH₂-O-C(O)-.

10. Compounds of formula IIIa 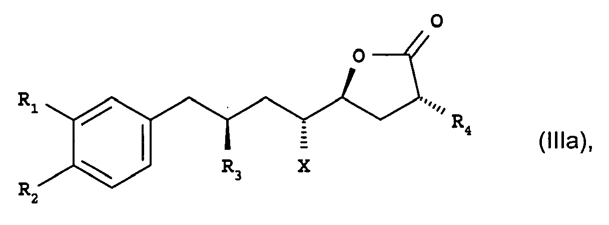 wherein R₁ R₂, R₃, R₄ and X are as defined in claim 1.

11. Compounds according to claim 10 comprising an embodiment wherein R₁ is 1-methoxyprop-3-yloxy, R₂ is methoxy, R₃ and R₄ are isopropyl and X is Cl, Br or I.

12. Compounds of formula Vlla 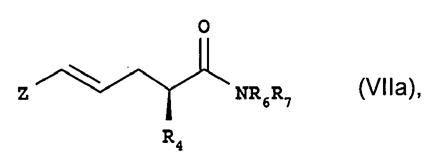 wherein R₄, R₆ and R₇ are as defined in claim 1, and Z is Cl, Br or I.

13. Compounds according to claim 13, comprising an embodiment wherein R₄ is 1-methyl ethyl, Z is Cl, and R₆ is methyl or ethyl, R₇ is methyl, ethyl or methoxy, or R₆ and R₇ together are tetramethylene, pentamethylene or CH(CH₂C₆H₅)CH₂-O-C(O)-.

14. Compounds of formula IVa 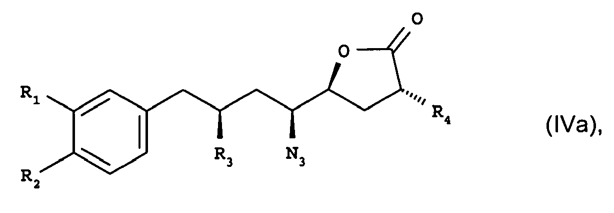 wherein R₁, R₂, R₃ and R₄ are as defined in claim 1.

## Revendications

1. Procédé de préparation de composés de formule la, 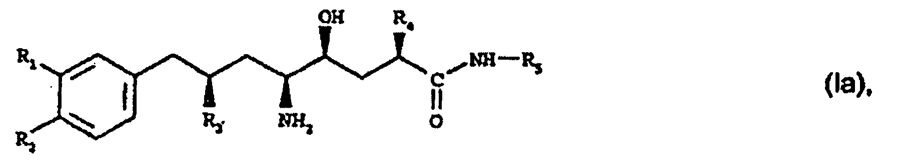 dans laquelle :
R₁ et R₂ représentent, indépendamment l'un de l'autre, un atome d'hydrogène, un groupe alkyle en C₁₋C₆, un groupe halogénoalkyle en C₁₋C₆, un groupe alcoxy en C₁₋C₆, un groupe C₁-C₆-alcoxy-C₁-C₆-alkyle, ou un groupe C₁₋C₆-alcoxy-C₁-C₆-alkyloxy, R₃ représente un groupe alkyle en C₁₋C₆, R₄ représente un groupe alkyle en C₁-C₆, et R₅ représente un groupe alkyle en C₁-C₆, un groupe hydroxyalkyle en C₁-C₆, un groupe C₁-C₆-alcoxy-C₁-C₆-alkyle, un groupe C₁₋C₆-alcanoyloxy-C₁₋C₆-alkyle, un groupe aminoalkyle en C₁₋C₆, un groupe C₁₋C₆-alkylamino-C₁₋C₆-alkyle, un groupe C₁₋C₆-dialkylamino-C₁₋C₆-alkyle, un groupe C₁₋C₆-alcanoylamido-C₁₋C₆-alkyle, un groupe HO(O)C-C₁₋C₆-alkyle, un groupe C₁₋C₆-alkylo-(O)C-C₁₋C₆-alkyle, un groupe H₂N-C(O)-C₁₋C₆-alkyle, un groupe C₁₋C₆-alkyl-HN-C(O)-C₁₋C₆-alkyle ou un groupe (C₁₋C₆-alkyl)₂N-C(O)-C₁₋C₆-alkyle, **caractérisé en ce que** :
a) un composé de formule IIa 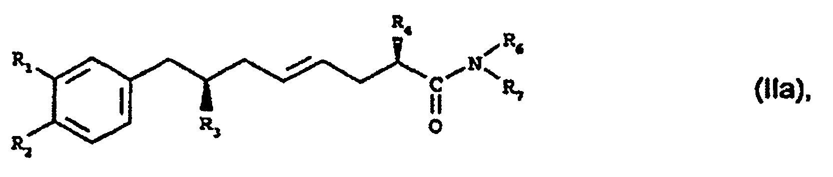 dans laquelle :
R₆ représente un groupe alkyle en C₁-C₆, R₇ représente un groupe alkyle en C₁-C₆ ou un groupe alcoxy en C₁-C₆, ou R₆ et R₇ représentent, conjointement, un groupe tétraméthylène, un groupe pentaméthylène, un groupe 3-oxa-1,5-pentylène ou un groupe -CH₂CH₂O-C(O)-, éventuellement substitué par un groupe alkyle en C₁-C₄, un groupe phényle ou un groupe benzyle, est mis à réagir avec un agent d'halogénation en présence d'eau et, éventuellement, d'un acide pour donner lieu à un composé de formule IIIa, 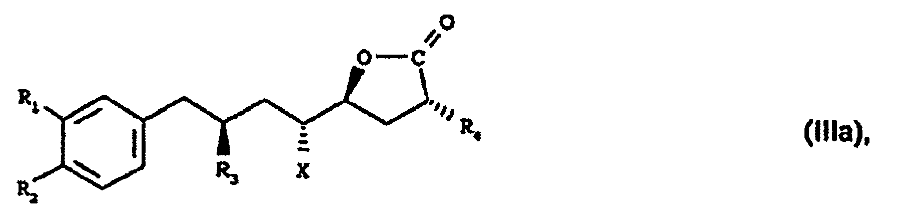
dans laquelle X représente un atome de chlore, un atome de brome ou un atome d'iode,
b) le composé de formule IIIa est mis à réagir avec un agent d'azidation pour donner lieu à un composé de formule IVa, 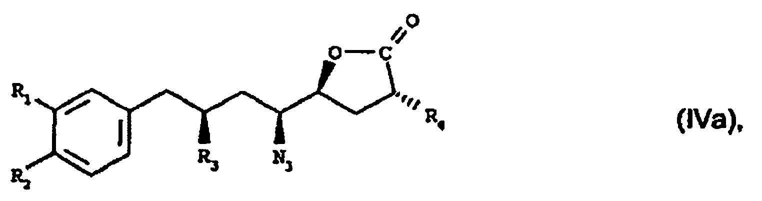
c) le composé de formule IVa est ensuite mis à réagir avec une amine de formule R₅-NH₂ pour donner lieu à un composé de formule Va, 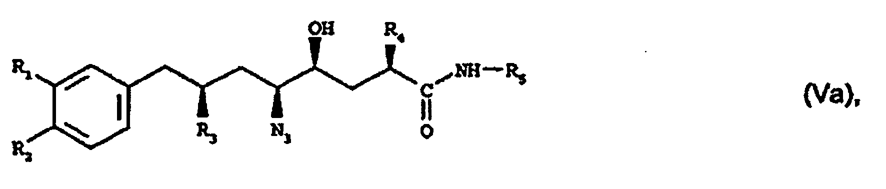 et
d) pour la préparation du composé de formule la, le groupe azide du composé de formule Va est réduit en groupe amine, puis les composés de formule la sont isolés, éventuellement en ajoutant un acide formant un sel.

2. Procédé selon la revendication 1, **caractérisé en ce que** R₁ représente un groupe alcoxy en C₁-C₄ ou un groupe C₁₋C₄-alcoxy-C₁₋C₄-alkyloxy, R₂ représente un groupe alcoxy en C₁-C₄, R₃ représente un groupe alkyle en C₁₋C₄, R₄ représente un groupe alkyle en C₁₋C₄ et R₅ représente un groupe H₂NC(O)-C₁₋C₆-alkyle éventuellement N-mono- ou N-di-C₁₋C₄-alkyl-substitué.

3. Procédé selon la revendication 2, **caractérisé en ce que** R₁ représente un groupe 1-méthoxyprop-3-yloxy et R₂ représente un groupe méthoxy.

4. Procédé selon la revendication 2, **caractérisé en ce que** R₃ et R₄ représentent chacun un groupe isopropyle.

5. Procédé selon la revendication 2, **caractérisé en ce que** R₅ représente un groupe H₂NC(O)-C₁₋C₆-alkyle.

6. Procédé selon la revendication 1, **caractérisé en ce que** R₁ représente un groupe méthoxy-C₂₋C₄-alkyloxy, R₂ représente un groupe méthoxy ou un groupe éthoxy, R₃ représente un groupe alkyle en C₂₋C₄, R₄ représente un groupe alkyle en C₂₋C₄, et R₅ représente un groupe H₂NC(O)-C₁₋C₆-alkyle.

7. Procédé selon la revendication 1, **caractérisé en ce que** R₁ représente un groupe 3-méthoxy-prop-3-yloxy, R₂ représente un groupe méthoxy, R₃ et R₄ représentent chacun un groupe 1-méthyléth-1-yle, et R₅ représente un groupe H₂NC(O)-[C(CH₃)₂]-CH₂-.

8. Composés de formule IIa 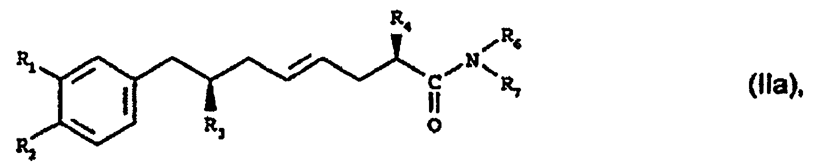 dans laquelle R₁, R₂, R₃, R₄, R₆ et R₇ présentent les significations indiquées dans la revendication 1.

9. Composés selon la revendication 8, **caractérisés en ce que** R₁ représente un groupe 1-méthoxy-prop-3-yloxy, R₂ représente un groupe méthoxy, R₃ et R₄ représentent un groupe isopropyle, et R₆ représente un groupe méthyle ou un groupe éthyle, R₇ représente un groupe méthyle, un groupe éthyle ou un groupe méthoxy, ou R₆ et R₇ représentent, conjointement, un groupe tétraméthylène, un groupe pentaméthylène ou un groupe -CH(CH₂C₆H₅)CH₂-O-C(O)-.

10. Composés de formule IIIa, 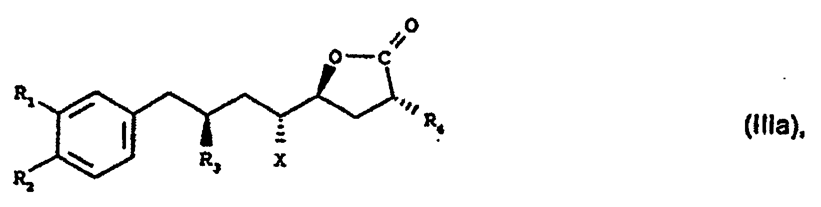 dans laquelle R₁, R₂, R₃, R₄ et X présentent les significations indiquées dans la revendication 1.

11. Composés selon la revendication 10, **caractérisés en ce que** R₁ représente un groupe 1-méthoxy-prop-3-yloxy, R₂ représente un groupe méthoxy, R₃ et R₄ représentent un groupe isopropyle, et X représente un atome de chlore, un atome de brome ou un atome d'iode.

12. Composés de formule VIIa, 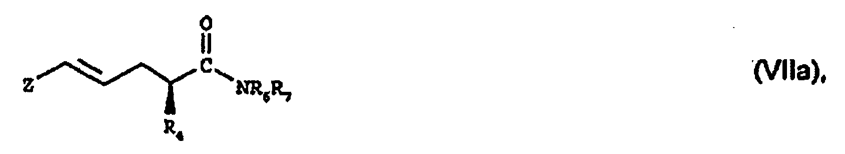 dans laquelle R₄, R₆ et R₇ présentent les significations indiquées dans la revendication 1, et Z représente un atome de chlore, un atome de brome ou un atome d'iode.

13. Composés selon la revendication 12, **caractérisés en ce que** R₄ représente un groupe 1-méthyléthyle, Z représente un atome de chlore, et R₆ représente un groupe méthyle ou un groupe éthyle, R₇ représente un groupe méthyle, un groupe éthyle ou un groupe méthoxy, ou R₆ et R₇ représentent, conjointement, un groupe tétraméthylène, un groupe pentaméthylène ou un groupe -CH(CH₂C₆H₅)CH₂-O-C(O)-.

14. Composés de formule IVa, 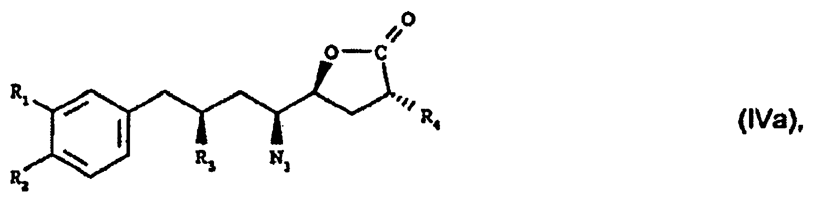 dans laquelle R₁, R₂, R₃ et R₄ présentent les significations indiquées dans la revendication 1.
